# EUROPEAN PATENT APPLICATION

(11) **EP 4 095 265 A1**
(43) Date of publication of application: **30.11.2022**
(21) Application number: 21305694.8
(22) Date of filing: 26.05.2021
(51) Int. Cl.: C12Q 1/6883

(54) **DETECTION OF KDM1A LOSS OF ACTIVITY FOR DIAGNOSING ENDOCRINE DISORDERS**

(71) Applicant: Université Paris-Saclay, 91190 Gif-sur-Yvette (FR); INSERM (Institut National de la Santé et de la Recherche Médicale), 75013 Paris (FR); Assistance Publique-Hôpitaux de Paris, 75004 Paris (FR); Centre Hospitalier de l'Université de Montréal, Montréal QC H2X 0A9 (CA)
(72) Inventor: BOULIGAND, Jérôme, 94700 MAISONS-ALFORT (FR); BOURDEAU, Isabelle, ST-LAMBERT, J4R 2W3 (CA); CHASSELOUP, FANNY, 94320 THIAIS (FR); LACROIX, André, MONT ROYAL, H3R 3A4 (CA); KAMENICKY, Peter, 75014 PARIS (FR)
(74) Representative: Regimbeau

(57) **Abstract**

The present invention proposes to detect genetic abnormalities (mutations and LOH) in the *KDM1A* gene of subjects, in order to diagnose a genetic predisposition to an endocrine disease and/or to an endocrine hyperplasia / or adenoma formation. Also, it proposes to treat patients with GIP-dependent PBMAH with Cushing's syndrome by restoring the expression of the *KDM1A* gene, by gene therapy.

## Description

### BACKGROUND OF THE INVENTION

Primary bilateral macronodular adrenal hyperplasia (PBMAH) is a benign lesion of the adrenal glands with a very slow growth rate. This hyperplasia is diagnosed in patients with clinical signs of cortisol excess, usually late in adult life - typically in the fifth or sixth decade of life.

In other patients, the disease is diagnosed without clinically apparent phenotype of hypercortisolism, when enlarged adrenal glands are incidentally detected on abdominal CT scans. Adrenal incidentalomas can be observed in 0.4-5% of the general population, and are bilateral in 10-15% of the cases, corresponding mostly to PBMAH. In fact, the exact prevalence of PBMAH in the general population is unknown and dependent of the diagnosis criteria.

PBMAH can be the cause of pituitary adrenocorticotropin-independent cortisol excess and Cushing's syndrome¹. The Cushing's syndrome is diagnosed in about 35% of these bilateral incidentalomas. Cushing's syndrome in PBMAH is often mild and insidious, even if serious forms can be encountered.

The Cushing's syndrome is responsible for many manifestations including central obesity, hypertension, cardiovascular disease, neuropsychiatric disorders, osteoporosis, glucose intolerance or diabetes. These many consequences lead to significant impairment of quality of life and, if untreated associated with increased mortality. Several studies suggest that despite the cure of cortisol excess, a significant morbidity persists and that even in cured patients life expectancy is reduced, due to long-term consequences of too long exposure to a cortisol excess (Clayton RN et al, 2016³⁵, Drougat et al, 2015³⁶).

It is therefore essential to identify strategies to reduce the duration of cortisol excess. Considering the rarity of Cushing's syndrome and the difficulties for its screening and diagnosis by non-specialized physicians, its early diagnosis is very challenging.

In this context, there is therefore an urgent need to identify diagnostic tools that enable to detect patients suffering from Cushing's syndrome before they have been exposed to cortisol excess in an irreversible manner.

It has been suggested that the Cushing's syndrome is triggered in some cases by genetic alterations that can help diagnose these patients earlier. For example, it has been shown that actors of the cAMP/protein kinase A (PKA) signaling pathway or genes causing an hereditary familial tumor syndrome including adenomatous polyposis coli gene (APC), menin (MEN1) and fumarate hydratase (FH) can favor or be responsible for the development of PBMAH, but these cases are very rare. Also, mutations in the *ARMC5* (Armadillo repeat containing protein 5) gene have been identified as a frequent cause of sporadic or familial PBMAH. Combining all the ARMC5 studies reported to date, a total of 29 germline and 32 somatic pathogenic mutations have been identified in approximately 25-35% of patients with apparently sporadic PBMAH (Drougat et al, 2015³⁶).

Inactivation of *ARMC5* in PBMAH follows the 'two-hit' model of a tumor suppressor gene responsible for a hereditary neoplasia syndrome. In the case of ARMC5, the model suggests that the loss of cell control in the adrenal cortex secondary to germline ARMC5 mutation leads to nodular hyperplasia development.

Despite *ARMC5* being the first gene discovered to be frequently involved in PBMAH, its germline and secondary somatic inactivating mutations were observed only in about one third of patients with PBMAH^{2.} In other words, the causes of two thirds of PBMAH adult cases are still not explained.

In a large proportion of patients with PBMAH and less frequently in those with unilateral adenoma, cortisol excess is driven by aberrant (ectopic or excessive) expression of several G-protein-coupled receptors (GPCR) in adrenal lesions³. Ectopic expression of the glucose-dependent insulinotropic polypeptide (GIP) receptor (GIPR) in PBMAH can be associated with an inverse cortisol rhythm with low fasting morning plasma cortisol concentrations which increase after food intake^{4,5}. The postprandial rise of cortisol secretion is induced by GIP, an incretin produced by intestinal K-cells following oral lipid, carbohydrates, or protein intake. Activation of the ectopic GIPR, functionally coupled to cAMP signaling, triggers adrenal cell proliferation and excessive steroid production. The molecular events leading to ectopic GIPR expression in the adrenocortical tissue are not well understood. Recently were reported somatic 19q13.32 microduplications containing the GIPR locus, rearranged with other chromosomal regions, in cortisol-secreting adenomas of two patients with GIP-dependent Cushing's syndrome⁸. However, the molecular pathogenesis of the ectopic GIPR expression in PBMAH remained to be elucidated.

If PBMAH in GIP-dependent Cushing's syndrome patients is also of genetic origin, the causal gene(s) of these diseases remained to be identified.

In this context, there is therefore an urgent need to better understand the molecular mechanisms responsible for the development of PBMAH in GIP-dependent Cushing's patients, so as to identify predisposition genetic factors that can explain the etiology of these diseases.

Knowing such genetic predisposition factors will favor an earlier diagnosis of PBMAH and of the GIP-dependent Cushing's syndrome that may be associated. It will help preventing the severe consequences of long-time exposure to cortisol excess to occur, and could therefore translate into efficient cures and prevention programs.

More generally, additional efforts are therefore needed to identify a reliable diagnostic marker that allows identifying genetic predisposition to endocrine tumors and to identify a reliable marker for prognosticating their progression into worse disease.

### DESCRIPTION OF THE INVENTION

To identify genetic predisposition factors, the present inventors have performed an exhaustive sequencing and copy-number analysis of blood and adrenal DNA from 12 (ce sera plus, on a recu 7 nouveau patients qui seront possiblement mutes, analyse en cours) patients with familial or sporadic GIP-dependent Cushing's syndrome with PBMAH, and from 16 (ce chiffre va aussi augmenter) patients with Cushing's syndrome with PBMAH without food induced cortisol secretion as controls. By doing so, they discovered that familial and sporadic GIP-dependent PBMAH with Cushing's syndrome is a genetic disease caused in 100% of cases by germline inactivating mutations of the lysine demethylase 1A (KDM1A), and with a systematic loss of heterozygosity of the second *KDM1A* locus in the adrenal lesions. More precisely, they identified germline heterozygous truncating or frameshift mutations in the *KDM1A* gene in all 12 patients with GIP-dependent PBMAH with Cushing's syndrome, and a recurrent deletion of the short arm of chromosome 1 harboring the KDM1A locus in the adrenal lesions of affected patients. None of the control subjects displayed any KDM1A germline or somatic alterations.

*KDM1A* encodes for a histone lysine demethylase, belonging to a larger family of such proteins⁷. Histone tails are subjected to covalent modifications that affect chromatin structure and the recruitment of regulatory factors consequently modifying transcription. Methylation of lysine residues can be associated with either activation or repression of transcription²³. KDM1A represses transcription by demethylating histone H3 on lysin 4 (H3K4me) a histone mark usually linked to active gene transcription ^{23,24}. In addition, KDM1A has also been shown to affect methylation of non-histone proteins involved in tumorigenesis such as p53, RB1 and STAT3⁷. Both mechanisms can be of importance in the pathogenesis of GIP-dependent Cushing's syndrome with PBMAH. Persistent histone methylation secondary to loss of KDM1A function can result in aberrant transcriptional activation, and absence of KDM1A interaction with oncogenic proteins that can lead to cell cycle dysregulation and consequently adrenal tumorigenesis.

The present inventors have shown that *in vitro* pharmacologic inhibition or silencing of KDM1A with siRNAs and also by using CRISPR/Cas9 technology resulted in an increase in GIPR transcripts and protein in human adrenocortical cells. Targeted exome sequencing did not detect any additional molecular events in genes known to be involved in adrenal tumorigenesis. Thus, loss of KDM1A function seems sufficient to induce aberrant GIPR expression in endocrine tissues, and in particular in pituitary or adrenal cells.

Steroidogenesis can be driven by the expression of ectopic receptors that are not expressed at significant levels in normal zona fasciculata cells, such as those for GIPR, beta-adrenergic receptors (b-AR), vasopressin (V2-V3-vasopressin receptor), serotonin (5-HT7 receptor), glucagon (GCGR) and probably angiotensin II (AT1R). It can also result from increased expression or increased coupling to steroidogenesis of eutopic receptors such as those for vasopressin (V1-vasopressin receptor), LH/human chorionic gonadotropin (LHCGR), or serotonin (5-HT4 receptor)³⁷.

The present inventors propose that *KDM1A* is a key epigenetic regulator of tissue-specific expression of GIP receptor and possibly of other receptors from the G-protein coupled receptor family in hormone-producing glands, and that its alteration leads to the development of aberrant overexpression of eutopic hormone receptors or expression of ectopic hormone receptors that lead to abnormal steroidogenesis. They also show that loss of expression of KDM1A is likely to be the initiating event that trigger the abnormal cell proliferation leading to the development of tissue lesions in adrenal and possibly in other endocrine tissues (notably in the adrenal glands and in the pituitary gland). These findings enable genetic testing and counselling of kindred of affected patients so as to detect earlier these diseases and avoid overexposure to aberrant levels of hormones.

The present inventors therefore propose to detect these genetic abnormalities (mutations and LOH) in the genome of subjects, in order to diagnose a genetic predisposition to an endocrine disease and/or to an endocrine hyperplasia / or adenoma formation. Also, they propose to treat patients with GIP-dependent PBMAH with Cushing's syndrome by restoring the expression of the *KDM1A* gene, by gene therapy.

### Definitions

By "endocrine disease", it is herein meant any disease or disorder due to excessive hormone (steroid or peptide) production by an endocrine tissue. This endocrine disease can be due to the overexpression of an eutopic hormone receptor in an endocrine tissue, or to the ectopic abnormal expression of an hormone receptor in an endocrine tissue. Endocrine tissues are for example thyroid, parathyroid, adrenal glands, pancreas, intestines and pituitary glands.

Endocrine disease may be accompanied by proliferative events and hyperplasia in the endocrine tissues, where overexpression or ectopic expression of the hormone receptor occurs. For example, the ectopic expression of GIPR in the adrenal tissue can promote PBMAH development. "Endocrine diseases" therefore encompass endocrine tissue lesions including adrenal hyperplasia, pituitary adenoma, adrenal myelolipoma and the like. In particular, the methods of the invention enable to diagnose (or to prognose a predisposition to) the disorders including (without being restricted to): Cushing syndrome, PBMAH associated to a Cushing syndrome, adrenal hyperplasia, pituitary adenoma, and adrenal myelolipoma.

By "hormone receptor", it is herein meant the GIP receptor LH/hCG receptor, the beta-adrenergic receptor, the angiotensin II receptor type 1, the glucagon receptor, the serotoninergic receptor, the vasopressin receptor, and other GPCR involved in hormone production (reviewed in El Gorayeb EJE 2015³⁷).

This endocrine disease is for example due to the overexpression or ectopic expression of GIPR in adrenal glands, which can lead on a one hand to the overproduction of cortisol and hence to the onset of a Cushing's syndrome. Cortisol is a steroid hormone that is produced by the adrenal glands. When released into the bloodstream, cortisol can act on many different parts of the body and can help the body respond to stress or danger, increase the body's metabolism of glucose, control blood pressure and reduce inflammation. The amount of cortisol produced by the adrenal glands is highly regulated by the pituitary gland to ensure the balance is correct.

This endocrine disease can also result from the overexpression or ectopic expression of GIPR in somatotroph cells of the pituitary gland thereby leading to somatotroph pituitary adenoma.

As used herein, the expression "biological sample" refers to any sample containing genomic DNA or mRNA or proteins from a subject. Said sample DNA may be contained in a solid tissue, in corporal fluids and/or excretions of said studied subject. Said fluid is for example buccal swab, sperm, blood, serum, plasma, or urine. In a preferred embodiment, the biological sample is a blood sample of said subject, bone marrow or spleen or skin biopsies, or any other cells. Indeed, such a blood sample may be obtained by a completely harmless blood collection from the subject and thus allows for a non-invasive diagnosis. The blood sample used in the method of the invention is preferably depleted of most, if not all erythrocytes, by common red blood cell lysis procedures. The detection is performed on the remaining blood cells, which are white blood cells (e.g., neutrophils, monocytes, lymphocytes, basophiles, etc.) and platelets.

As used herein, the term "subject" refers to any mammal, preferably a human. Said subject may be a currently healthy individual. Yet, the method of the invention is particularly useful for testing a subject that is predisposed to developing an endocrine disease, including, but not restricted to, a Cushing's syndrome or an endocrine lesion, for example because it belongs to a family in which one member at least is concerned by such a disease. In that case, the method of the invention enables to confirm that said subject will develop or is predisposed for developing such as disease. Said subject has for example at least one parent (brother, sister, mother, father, grand-mother, grand-father, etc.) carrying the KDM1A mutation of the invention.

By "homologous", it is herein meant that the defined sequences encode the same proteins but, due to codon degeneracy, are not identical and have sequence similarity. The term "sequence similarity", in all its grammatical forms, refers to the degree of identity or correspondence between the said nucleic acid sequences. In a preferred embodiment, the homologous genomic region to be detected by the methods of the invention has a nucleotide sequence sharing at least 80% identity, preferably 90% identity, more preferably 95% identity with SEQ ID NO:1 (DNA of the *KDM1A* gene corresponding to NG_047129.1) or SEQ ID NO:2 (mRNA of the KDM1A gene corresponding to NM_001009999.3, encoding the protein of SEQ ID NO:3 corresponding to NP_001009999.1).

For the purpose of the present invention, the "percentage of identity" between two nucleic acid sequences is intended to refer to a percentage of nucleotides which are identical between the two sequences obtained after the best alignment. This percentage is purely statistical and the differences between the two sequences are distributed randomly and throughout their length. Sequence comparisons between two nucleic acids are traditionally carried out by comparing these sequences after having optimally aligned them, said comparison being carried out by segment or by "window of comparison" in order to identify and compare local regions of sequence similarity. The optimal alignment of the sequences for comparison can be produced, besides manually, by means of the global homology algorithm of Needleman and Wunsch (1970) [J. Mol. Biol. 48:443. The percentage of identity is calculated by determining the number of identical positions for which the nucleotide or the amino acid residue is identical between the two sequences, dividing this number of identical positions by the total number of positions and multiplying the result obtained by 100 so as to obtain the percentage of identity between these two sequences. For example, the needle program available on the site ebi.ac.uk, may be used, the parameters used being those given by default (in particular for the parameters "Gap open": 10, and "gap extend":0.5; the matrix chosen being, for example, the "BLOSUM 62" matrix proposed by the program), the percentage of identity between the two sequences to be compared being calculated directly by the program.

By "invalidating mutation" it is herein encompassed any mutation at the gene level that leads to a lack of or lowered expression of the KDM1A protein or any mutation at the protein level that results in an abnormal or reduced activity of the KDM1A protein. It corresponds to any modification in the sequence of the original nucleic acid sequence of SEQ ID NO:1 or in the protein sequence of SEQ ID NO:3. These mutations comprise small-scale mutations, or large scale mutations. Small scale mutations are those affecting a gene in one or a few nucleotides, including point mutations, insertions or deletions of one or more extra nucleotides in the DNA. Point mutations can be silent, missense and nonsense mutation. Large scale mutation in the genomic structure, such as gene duplications, deletions, or mutations whose effect is to juxtapose previously separate pieces of DNA, potentially bringing together separate genes to form functionally distinct fusion genes. These last mutations include chromosomal translocations, interstitial deletions, chromosomal inversions and loss of heterozygosity. At the protein level, these mutations can be a punctual mutation modifying the activity of the enzyme, or result in an abnormal truncation.

KDM1A encodes for a histone lysine demethylase of SEQ ID NO:3, belonging to a larger family of such proteins⁷. Histone tails are subjected to covalent modifications that affect chromatin structure and the recruitment of regulatory factors consequently modifying transcription. Methylation of lysine residues can be associated with either activation or repression of transcription²³. KDM1A represses transcription by demethylating histone H3 on lysin 4 (H3K4me), a histone mark usually linked to active gene transcription *^{23,24}*.

The invalidating mutation(s) of the invention preferably result in the absence of expression of the KDM1A protein or in the expression of a truncated KDM1A protein, said truncated protein having lost its histone demethylase function, more precisely, its ability to demethylate histone H3 on lysin 4. It can also result in the expression of a non-functional enzyme, i.e., an enzyme having lost its histone demethylase function.

The DNA invalidating mutations encompassed by the present invention preferably affect the level of expression and/or the activity of the KDM1A protein of SEQ ID NO:3 as compared to normal control samples from healthy subject (blood or endocrine tissue samples). They lower the quantity of KDM1A which is normally expressed, or produce a truncated protein or a non-functional protein, in all tissues (if germline) and specifically in endocrine tissues (if somatic).Typical techniques for detecting the presence of a mutation may include restriction fragment length polymorphism, hybridization techniques, DNA sequencing, exonuclease resistance, microsequencing, solid phase extension using ddNTPs, extension in solution using ddNTPs, oligonucleotide ligation assays, methods for detecting single nucleotide polymorphisms such as dynamic allele-specific hybridization, ligation chain reaction, mini-sequencing, DNA "chips", allele-specific oligonucleotide hybridization with single or dual-labelled probes merged with PCR or with molecular beacons, and others.

Advantageously, the alteration is detected on the cDNA or DNA of the *KDM1A* gene by either PCR and sequencing, SNP-array or CGH, all these technologies being well known for the skilled person.

In molecular biology and bioinformatics, a SNP array is a type of DNA microarray which is used to detect polymorphisms within a population. The basic principles of SNP array are the same as the DNA microarray. These are the convergence of DNA hybridization, fluorescence microscopy, and solid surface DNA capture. The three mandatory components of the SNP arrays are: i) the array that contains immobilized nucleic acid sequences or target; ii) one or more labelled Allele specific oligonucleotide (ASO) probes; and iii) a detection system that records and interprets the hybridization signal.

Comparative genomic hybridization (CGH) is a molecular cytogenetic method of screening a sample for genetic changes. The method is based on the hybridization of fluorescently target DNA (frequently fluorescein (FITC)) and normal DNA (frequently rhodamine or Texas Red) to normal human metaphase preparations. Using epifluorescence microscopy and quantitative image analysis, regional differences in the fluorescence ratio of gains/losses vs. control DNA can be detected and used for identifying abnormal regions in the genome.

In particular, characterizing the presence of *KDM1A* gene as proposed in the invention can be performed by PCR (primers are listed below).

As used herein, the "under-expression" or "reduced expression" of a polypeptide occurs when the transcription and/or the translation of the gene is affected by the mutation, leading to an expression level in a biological sample that is lower than the standard error of the assay employed to assess expression, and is preferably at least 20% inferior to the normal level of expression of said gene, preferably at least 50% inferior to the normal level of expression of said gene, and most preferably at least 100% inferior to the normal level of expression of said gene. In other words, a reduced expression of the KDM1A protein is detected if the amount of protein as detected by conventional means (e.g., by Western Blot) is at least 20%, preferably at least 50%, preferably at least 90% inferior to the amount of the same protein in control samples.

Therefore, the method of the invention may comprise comparing the level of expression of the *KDM1A* gene in a biological sample from a subject with its expression level in a control (i.e., normal expression level). A significantly lower level of expression of said gene in the biological sample of a subject as compared to the normal expression level is an indication that the patient may develop an endocrine disease.

As used herein, a "control" corresponds preferably to a control sample comprising cells from a healthy subject or from a subject that does not suffer from an endocrine disease. More preferably, said control sample corresponds to peripheral blood leukocytes (PBL) of a healthy subject or granulocytes or platelets or any other kind of cells, e.g., cells of an endocrine tissue. The "normal" level of expression of a gene corresponds to the level of expression of said gene in such control sample. Also, the "normal" activity or expression of a protein corresponds to the activity or expression of said protein in such control sample.

The expression of the KDM1A protein can be detected by any conventional means, for example by Western Blot, enzyme immunoassay (EIA), radioimmunoassay (RIA), and enzyme linked immunoabsorbant assay (ELISA).

The function of the KDM1A protein can be detected by any conventional means, e.g., by evaluating its ability to demethylate histone H3 on lysin 4.

Polyclonal antibodies can be prepared by immunizing a suitable animal, such as mouse, rabbit or goat, with the targeted protein (KDM1A) or a fragment thereof (e.g., at least 10 or 15 amino acids of the KDM1A protein). The antibody titer in the immunized animal can be monitored over time by standard techniques, such as with an ELISA using immobilized polypeptide. At an appropriate time after immunization, e.g., when the specific antibody titers are highest, antibody producing cells can be obtained from the animal and used to prepare monoclonal antibodies (mAb) by standard techniques, such as the hybridoma technique originally described by KOHLER and MILSTEIN, the human B cell hybridoma technique, the EBV- hybridoma technique or trioma techniques. The technology for producing hybridomas is well known. Hybridoma cells producing the desired monoclonal antibody can be detected by screening the hybridoma culture supernatants for antibodies that bind the polypeptide of interest, e.g., using a standard ELISA.

In the context of the present invention, an antibody is said to "recognize" or "bind" a peptide having a define sequence if said antibody has an affinity constant Kₐ (which is the inverted dissociation constant, i.e. 1/K_{d}) higher than 10⁶ M⁻¹, preferably higher than 10⁷ M⁻¹, more preferably higher than 10⁹ M⁻¹ for said peptide. Also, in the context of the present invention, an antibody is said to "specifically bind" or to "specifically recognize" a peptide if said antibody has an affinity constant Kₐ higher than 10⁶ M⁻¹, preferably higher than 10⁷ M⁻¹, more preferably higher than 10⁹ M⁻¹ for said peptide and has an affinity constant Kₐ lower than 10⁴ M⁻¹ for all the other peptide.

As used herein, "primers" designate isolated nucleic acid molecules that can specifically hybridize or anneal to 5' or 3' regions of a target genomic region (plus and minus strands, respectively, or vice-versa). In general, they are from about 10 to 30 nucleotides in length and anneal at both extremities of a region containing about 50 to 200 nucleotides in length. Under appropriate conditions and with appropriate reagents, such primers permit the amplification of a nucleic acid molecule comprising the nucleotide sequence flanked by the primers. As they have to be used by pairs, they are often referred to as "primers pair" or "primers set".

As used herein, "probes" are molecules that are capable of specifically hybridizing a genomic region of interest (e.g., of SEQ ID NO:1). They are useful to highlight the presence of said genomic region in biological samples. These probes may comprise at least one non-natural nucleotide, e.g., a peptide nucleic acid (PNA), a peptide nucleic acid having a phosphate group (PHONA), a bridged nucleic acid or locked nucleic acid (BNA or LNA), and a morpholino nucleic acid. Non-natural nucleotides also include chemically modified nucleic acids or nucleic acid analogs such as methylphosphonate-type DNA or RNA, phosphorothioate-type DNA or RNA, phosphoramidate-type DNA or RNA, and 2 -O-methyl-type DNA or RNA.

For certain uses, the probes and primers of the invention may be labeled - directly or indirectly - with a detectable label. Such label may be of any kind, depending on the experiment which is to be performed. Such label may be a radioactive isotope (such as ³²P, ³³P, ³⁵S, ³H or ¹²⁵I, or a nonradioactive entity which is selected from ligands (such as biotin, avidin or streptavidin), dioxygenin, haptens, colorants and luminescent agents (such as radioluminescent, chemiluminescent, bioluminescent, fluorescent or phosphorescent agents). Preferably, 6-carboxyfluorescein (FAM) and tetramethylrhodamine (TAMRA) are used. Non-labeled polynucleotide sequences may also be used, directly, as a probe or primer, for example in PCR-based processes (e.g., in quantitative PCR).

"Specific hybridization" is observed when a define molecule does not hybridize with any other genomic region than its target genomic region. Preferably, it hybridizes with its target region in high stringency conditions, i.e., when the temperature and ionic strength conditions are chosen so as to allow the hybridization between two complementary DNA fragments. By way of illustration, high stringency conditions can be as follows. The DNA-DNA or DNA-RNA hybridization is carried out in two steps: (1) prehybridization at 42°C for 3 hours in phosphate buffer (20 mM, pH 7.5) containing 5*SSC (1*SSC corresponds to a 0.15 M NaCl+0.015 M sodium citrate solution), 50% of formamide, 7% of sodium dodecyl sulfate (SDS), 10*Denhardt's, 5% of dextran sulfate and 1% of salmon sperm DNA; (2) actual hybridization for 20 hours at a temperature dependent on the size of the probe (i.e. 42°C. for a probe of size>100 nucleotides), followed by two 20-minute washes at 20°C. in 2*SSC+2% SDS and one 20-minute wash at 20°C. in 0.1*SSC+0.1% SDS. The final wash is carried out in 0.1*SSC+0.1% SDS for 30 minutes at 60°C for a probe of size>100 nucleotides. The high stringency hybridization conditions described above for a polynucleotide of defined size will be adjusted by those skilled in the art for oligonucleotides of greater or smaller size, according to the teaching of Sambrook et al., 1989.

"Specific amplification" of a target region (e.g., of SEQ ID NO:1) is observed when primers specifically hybridizing the 5' or 3' regions surrounding said target region are used. Such a specific amplification may also be observed when primers specifically hybridizing within the genomic region of interest are used.

As used herein, the terms *"in vitro"* and *"ex vivo"* are equivalent and refer to studies or experiments that are conducted using biological components (e.g., cells or population of cells) that have been isolated from their usual host organisms (e.g., animals or humans). Such isolated cells can be further purified, cultured or directly analyzed to assess the presence of the mutation of the invention. These experiments can be for example reduced to practice in laboratory materials such as tubes, flasks, wells, eppendorfs, etc. In contrast, the term *"in vivo"* refers to studies that are conducted on whole living organisms.

Within the scope of the present invention, by "nucleic acid" is meant mRNA, genomic DNA or cDNA derived from mRNA.

As used herein, the term "kit" refers to any system for delivering materials. In the context of reaction assays, it includes systems that allow the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contains a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

### Methods for predicting predisposition to endocrine diseases

It was discovered that the loss of function of the histone demethylase KDM1A protein in the adrenal and pituitary tissues results in ectopic expression of GIPR in the adrenal gland. This epigenetic regulation of tissue-specific expression of GIP receptor, and of any other GPCRs, has never been demonstrated.

More precisely, the present inventors herein showed that a lack of or a diminished expression of the gene *KDM1A* in germinal and endocrine tissues triggers the abnormal over or eutopic expression of GPCRs involved different endocrine diseases such as the GIP-dependent Cushing's syndrome with PBMAH). They therefore propose to use this lack of or reduced expression and/or activity of the KDM1A protein as a genetic predisposing marker, that should be analyzed in order to improve early diagnosis of endocrine diseases in familial screening.

In a first aspect, the present invention therefore relates to an *in vitro* method for identifying a genetic predisposition to an endocrine disease in a subject in need thereof, said method comprising the step of analyzing a biological sample from said subject so as to detect the presence of a germline invalidating mutation in the *KDM1A* gene and/or an abnormal expression or activity of the KDM1A protein in said sample.

More precisely, this method can comprise the following steps:
(i) detecting the presence of a germline invalidating mutation in the *KDM1A* gene having the SEQ ID NO:1 or SEQ ID NO:2, or in a fragment thereof, in the biological sample of said subject, and/or
(ii) detecting the abnormal expression or activity of the KDM1A protein having the SEQ ID NO:3 in the biological sample of said subject, for example by Western Blot.

If a heterozygous germline invalidating mutation is detected in the *KDM1A* gene or in a fragment thereof, or if an abnormal expression or activity of the KDM1A protein is detected, this indicates that the subject is predisposed to develop an endocrine disease, for example a Cushing syndrome, a PBMAH associated to a Cushing syndrome, adrenal hyperplasia, pituitary adenoma, or adrenal myelolipoma.

In the method of the invention, the terms "biological sample", "subject", "invalidating mutation", and "fragment" are as defined above. Said method may involve primers, probes and antibodies as defined above, and any of the above-mentioned technologies.

Preferably, said biological sample is a blood sample.

The genomic region of SEQ ID NO:1 is found on chromosome 1, more precisely on the short arm of the chromosome 1. This genomic region includes the *KDM1A* gene. In a preferred embodiment, the analyzing step of the method of the invention is performed on the whole genomic DNA of the subject, for example by using next-generation sequencing after capturing the regions of interest by probes. The mutations detected by this method are either single nucleotide variations (SNV), insertion/deletions (INDEL) or copy number variations (CNV). In a more preferred embodiment, this analysis is performed on chromosome 1, even more preferably on the short arm of chromosome 1.

In a preferred embodiment, the germline invalidating mutation detected in the methods of the invention in the *KDM1A* gene leads to a mutation is chosen in the group consisting of : deletions, insertions, point mutations such as mutations affecting splice sites, truncating mutations, frameshift mutations, missense mutation and nonsense mutations.

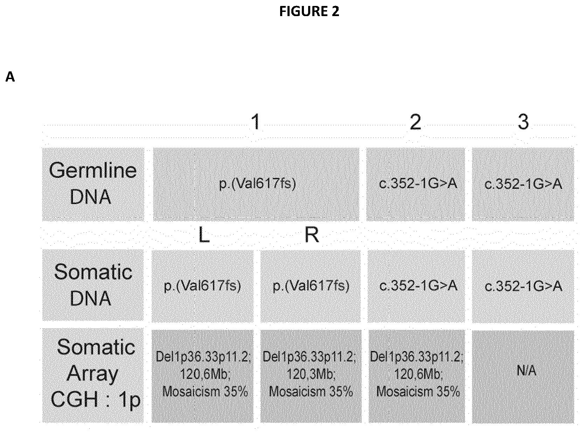

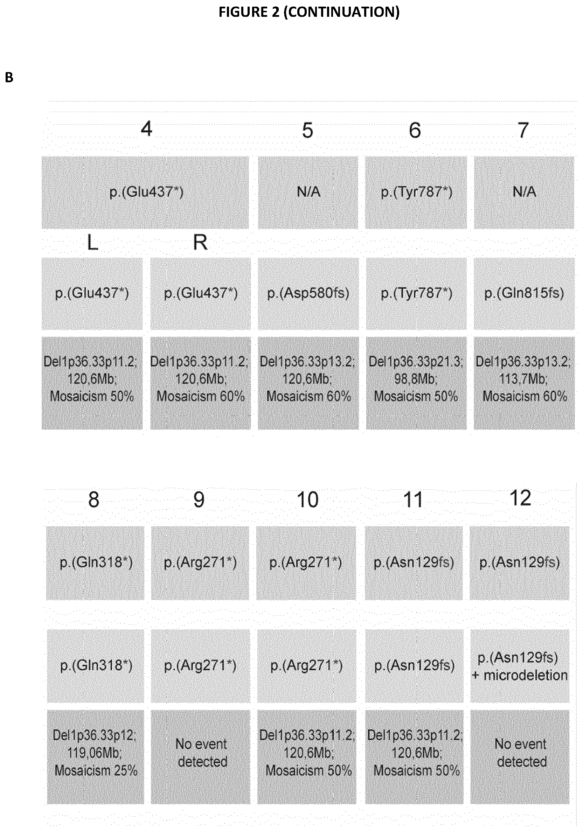

As shown in the examples below, the inventors have identified 5 germline frameshift mutations, 5 germline nonsense mutations and one germline splicing mutation in ten patients suffering from PBMAH and GIP-dependent Cushing's syndrome (figure 2).

In a preferred embodiment, said germline invalidating mutation in the *KDM1A* gene is chosen in the group consisting of: c.1848dupG, c.352-1G>A, c.1309G>T, c.1737_1738insGA, c.2361T>G, c.2441_2445del, c.952C>T, c.811C>T, and c.386delA. Said germline mutation can also be a deletion of part of or the whole *KDM1A* locus.

In a preferred embodiment, said germline invalidating mutation in the KDM1A gene is a deletion of the whole *KDM1A* locus, occurring on at least one allele of the chromosome 1, preferably on only one allele of chromosome 1.

The presence of the germline invalidating mutation of the invention is preferably identified when the NGS ratio between the mutated allele is of 50% in a non-endocrine tissue sample such as blood sample.

In a preferred embodiment, said germline invalidating mutation leads to a protein mutation chosen in the group consisting of: (p.Va1617GlyfsTer9), p.(Val617is), , p.Glu437, p(Tyr787Ter), p.(Gln317), p.(Asn129fs), (p.Gln815AspfsTer14), (p.Asp580GlufsTer11), (p.Gln318Ter), (p.Asn129ThrfsTer80) and p.(Arg271Ter).

| **Patient no.** | **HGVS Nomenclature: DNA** NM_001009999.3 | **HGVS Consequence** | **Annotation** | **MAF in gnomAD (%)** | ***VarSome ACMG classication*** |
|---|---|---|---|---|---|
| #1 | c.1848dupG | (p.Val617GlyfsTer9) | Frame shift | NR | Pathogenic |
| #2 | c.352-1G>A | Intronic | Splice acceptor | NR | Pathogenic |
| #3 | c.352-1G>A | Intronic | Splice acceptor | NR | Pathogenic |
| #4 | c.1309G>T | (p.Glu437Ter) | Stop gained | NR | Pathogenic |
| #5 | c.1737_1738insGA | (p.Asp580GlufsTer11) | Frameshift | NR | Pathogenic |
| #6 | c.2361T>G | (p.Tyr787Ter) | Stop gained | NR | Pathogenic |
| #7 | c.2441_2445del | (p.Gln815AspfsTer14 ) | Frameshift | NR | Pathogenic |
| #8 | c.952C>T | (p.Gln318Ter) | Stop gained | NR | Pathogenic |
| #9 | c.811C>T | (p.Arg271Ter) | Stop gained | NR | Pathogenic |
| #10 | c.811C>T | (p.Arg271Ter) | Stop gained | NR | Pathogenic |
| #11 | c.386delA | (p.Asn129ThrfsTer80) | Frame shift | NR | Pathogenic |
| #12 | c.386delA | (p.Asn129ThrfsTer80) | Frame shift | NR | Pathogenic |

These germline mutations can be detected in any biological sample containing cells including genomic DNA or mRNA or proteins from the subject to be tested. Preferably, said biological sample is a blood sample.

The analysing step aiming at detecting heterozygous germline invalidating mutations that may impair or lower the expression of the KDM1A protein is preferably performed in a blood sample.

To be useful in the method of the invention, it is sufficient that the invalidating mutation is heterozygous, i.e., found only on one of the two alleles of chromosome 1.

In a particular embodiment, the invalidating mutation to be detected is included in the group consisting of : deletions, insertions and point mutations such as mutations affecting splice sites, missense mutation and nonsense mutations, preferably missense mutation and nonsense mutations.

Said deletion or insertion preferably results in the absence of expression of the KDM1A protein or in an under-expression of the KDM1A protein, or in the expression of a truncated or non-functional KDM1A protein, said truncated or non-functional protein having lost its histone demethylase function.

Said missense mutation is preferably located in the open reading frame encoding the KDM1A protein.

Said nonsense mutation preferably results in the introduction of a stop mutation in the open reading frame encoding the KDM1A protein.

Preferably, said mutations are truncating or frameshift mutations that impair the lysine demethylase activity of KDM1A, more precisely, its ability to demethylate histone H3 on lysin 4.

To perform the method of the invention, it is sufficient to detect the presence of an invalidating mutation in a fragment of the genomic region of SEQ ID NO:1 or of SEQ ID NO:2. Said fragment can contain for example at least 10, more preferably at least 20, even more preferably at least 30 consecutive nucleotides of SEQ ID NO:1 or of SEQ ID NO:2.

The invalidating mutation of the invention can be detected by analysing the DNA structure of the *KDM1A* gene or of fragments thereof, e.g., by cytogenetic techniques such as comparative genomic hybridization array, or virtual karyotyping with SNP microarrays, by next-generation sequencing or by quantitative PCR.

In the examples below, the analysis of the *KDM1A* gene has been performed by using next-generation sequencing (Ilumina method) after capturing the regions of interest by probes (Agilent Sureselect XT method). The mutations detected by this method are either single nucleotide variations (SNV), insertion/deletions (INDEL) or copy number variations (CNV). The identified anomalies have been confirmed by 2 techniques: PCR and Sanger sequencing for SNV/INDEL and quantitative PCR (qPCR) for CNV. Preferably, NGS will therefore be used in step ii) of the invention (optionally confirmed by PCR).

According to the results obtained by the inventors, the absence of expression or the under-expression of the KDM1A protein or the expression of a truncated or non-functional KDM1A protein as disclosed previously may be associated with an endocrine disease.

Alternatively, the invalidating mutation of the invention can thus be detected by analyzing the level and structure of the mRNA of the *KDM1A* gene in said biological sample. A significantly lower level of the mRNA of said gene, or a significantly shorter mRNA of said gene in the biological sample of a subject as compared to the mRNA of SEQ ID NO:2 is an indication that the patient may develop an endocrine disease. In this embodiment of the invention, the method of the invention thus requires the analysis of the expression of mRNA transcript of SEQ ID NO:2, or of mRNA precursors of the *KDM1A* gene, or of fragments thereof as defined above.

Such analysis can be performed by preparing mRNA/cDNA from a biological sample from a subject, and hybridizing the mRNA/cDNA with a reference polynucleotide. The prepared mRNA/cDNA can be used in hybridization or amplification assays that include, but are not limited to, Southern or Northern analyses, polymerase chain reaction analyses, such as quantitative PCR (TAQMAN), and probes arrays such as GENECHIP^{™} DNA Arrays (AFFYMETRIX) or RNA-sequencing. The analysis of the expression level of mRNA transcribed from the *KDM1A* gene may involve the process of nucleic acid amplification, e.g., by RT-PCR, ligase chain reaction, self-sustained sequence replication, transcriptional amplification system, Q-Beta Replicase, rolling circle replication or any other nucleic acid amplification method, followed by the detection of the amplified molecules using techniques well known to those of skill in the art.

As previously mentioned, mutations in the *KDM1A* gene may trigger the absence of expression or the under-expression of the KDM1A protein or the expression of a truncated or non-functional protein. In a preferred embodiment of the invention, the method of the invention thus requires the analysis of the expression of the *KDM1A* protein of SEQ ID NO:3. In this case, the presence of the invalidating mutation of the invention is detected by analyzing the expression level of the proteins translated from the *KDM1A* gene. Such analysis can be performed using an antibody (e.g., a radiolabeled, chromophore-labeled, fluorophore-labeled, or enzyme-labeled antibody), an antibody derivative (e.g., an antibody conjugate with a substrate or with the protein or ligand of a protein of a protein/ligand pair (e.g., biotin-streptavidin), or an antibody fragment (e.g., a single-chain antibody, an isolated antibody hypervariable domain, etc.) which recognize specifically the KDM1A protein (SEQ ID NO:3). Said analysis may involve a variety of techniques well known by one of skill in the art including (but not limited to) enzyme immunoassay (EIA), radioimmunoassay (RIA), Western blot analysis and enzyme linked immunoabsorbant assay (ELISA).

In one embodiment, the method of the invention detects an abnormal expression or activity due to a reduced expression of the KDM1A protein of SEQ ID NO:3 as compared to a control sample, or due to the expression of a truncated non-functional KDM1A protein or due to the presence of another invalidating mutation affecting the KDM1A enzymatic activity.

If the subject has a lower expression of the *KDM1A* gene, due to the presence of an invalidating mutation, or if the subject expresses a mutated KDM1A protein that displays a lower histone demethylase activity, then aberrant expression of G protein receptors can be induced in adrenal cells, triggering hyperplasia and/or aberrant steroidogenesis.

Alternatively, the invalidating mutation of the invention can be detected by measuring the histone demethylase activity of the KDM1A protein in a biological sample of the tested subject, more precisely, its ability to demethylate histone H3 on lysin 4. If this activity is reduced as compared to a control sample, then the subject is diagnosed to be predisposed to develop an endocrine disease, for example a Cushing syndrome, a PBMAH associated to a Cushing syndrome, adrenal hyperplasia, pituitary adenoma, or adrenal myelolipoma.

Preferably, the method of the invention uses the primers of SEQ ID NO:4-5.

### Methods for diagnosing endocrine diseases

As explained above, an altered expression of the *KDM1A* gene and/or the activity of the KDM1A protein, when assessed in a blood sample of a subject, is indicative that said subject is predisposed to suffer from an endocrine disease in the future. This endocrine disease may develop as soon as a second mutation occurs in one of the endocrine tissues, e.g., in adrenal or pituitary tissues.

In a second aspect, the present invention thus relates to an *in vitro* method for the early diagnosis of an endocrine disease in a subject in need thereof, said method comprising the steps of:
- detecting the presence of a heterozygous germline invalidating mutation in the *KDM1A* gene or in a fragment thereof, or detecting the abnormal expression and/or activity of the KDM1A protein, in a first biological sample of said subject,
   and
- detecting the presence of another invalidating mutation in the *KDM1A* gene or in a fragment thereof, in a specimen of endocrine tissue of the same subject.

In a preferred embodiment, said first biological sample is blood or any sample containing germline DNA.

Said specimen of endocrine tissue is for example obtained by biopsy or after therapeutic surgery.

The detection of a germline invalidating mutation on one allele of the *KDM1A* gene, as identified in the blood sample, and the detection of a somatic invalidating mutation affecting the other allele of *KDM1A* in the cells of the endocrine tissue, indicates that the subject is developing an endocrine disease, for example a Cushing syndrome, a PBMAH associated to a Cushing syndrome, adrenal hyperplasia, pituitary adenoma, or adrenal myelolipoma.

Also, the detection of an abnormally low level or reduced activity of the KDM1A protein in the blood sample of a subject, and the detection of a somatic invalidating mutation affecting the one allele of the *KDM1A* gene in the cells of the endocrine tissue indicates that the subject is developing an endocrine disease, for example a Cushing syndrome, a PBMAH associated to a Cushing syndrome, adrenal hyperplasia, pituitary adenoma, or adrenal myelolipoma.

The two mutations (germline and somatic) can be identical or different. Usually, the two mutations occur independently and are therefore different.

It is also possible to diagnose the development of an endocrine disease directly by detecting a double-mutation in the two alleles of the *KDM1A* gene in one endocrine tissue.

Alternatively, an early diagnosis of an endocrine disease can thus be obtained on a single sample of a subject, namely, on a specimen of endocrine tissue, by :
- detecting the presence of invalidating mutations on the two alleles of the *KDM1A* gene in said tissue,
   or
- detecting an abnormal low expression and/or reduced activity of the KDM1A protein in said tissue.

All the technics disclosed above for the predisposition predicting method of the invention apply, *mutatis mutandis,* to the diagnostic methods of the invention.

In particular, the germline or somatic invalidating mutations in the *KDM1A* gene can be found in SEQ ID NO:1 or SEQ ID NO:2 representing the DNA and the mRNA of the *KDM1A* gene, respectively. They can be detected by any molecular technic known in the art, and, in particular, using next-generation sequencing after capturing the regions of interest by probes. In a more preferred embodiment, this analysis is performed on chromosome 1, even more preferably on the short arm of chromosome 1.

Also, the expression of the KDM1A protein can be measured by detecting the expression level of the KDM1A protein of SEQ ID NO:3, for example by Western Blot. The activity of the enzyme can be detected by any conventional means.

The DNA mutations are preferably chosen in the group consisting of: c.1848dupG, c.352-1G>A, c.1309G>T, c.1737_1738insGA, c.2361T>G, c.2441_2445del, c.952C>T, c.811C>T, and c.386delA. It can be a deletion of part or the whole KDM1A locus.

The DNA invalidating mutations encompassed by the present invention preferably affect the level of expression and/or the activity of the KDM1A protein of SEQ ID NO:3. They preferably lower the quantity of KDM1A which is normally expressed, or produce a truncated protein or a non-functional protein, in all tissues and specifically in endocrine tissues. They for example induce a protein mutation chosen in the group consisting of: (p.Val617G1yfsTer9), p.(Val617is), p.Glu437, p(Tyr787Ter), p.(Gln317), p.(Asn129fs), (p.Gln815AspfsTer14), (p.Asp580GlufsTer11), (p.Gln318Ter), (p.Asn129ThrfsTer80) and p.(Arg271Ter).

The presence of said invalidating mutations or the detection of an abnormally low expression and/or reduced activity of the KDM1A protein of SEQ ID NO:3 in a sample of endocrine tissue indicates that said subject is developing or is likely to develop very soon an endocrine disease, for example a Cushing syndrome, a PBMAH associated to a Cushing syndrome, adrenal hyperplasia, pituitary adenoma, or adrenal myelolipoma.

In particular, said endocrine disease will be diagnosed if the NGS ratio of the mutated alleles in said endocrine tissue is superior to 50%.

Preferably, the diagnostic methods of the invention use the primers of SEQ ID NO:4-5.

### Methods for treating endocrine diseases

The presence of such germline invalidating mutations and/or reduced activity indicates that the tested subject is developing an endocrine disease or on the process of developing an endocrine disease. The methods of the invention therefore enable to diagnose very early that an endocrine disease is developing.

Once this disease is diagnosed, appropriate treatments can be performed, based on the discovery of the present inventors.

In particular, it could be interesting to use gene therapy in order to restore normal expression of the KDM1A protein in the endocrine tissue at least. This gene therapy could be based on the local delivery and expression of the normal *KDM1A* gene in the cells of the endocrine tissue, by any conventional vector. In a preferred embodiment, this vector would be a replication defective recombinant virus encoding the *KDM1A* gene placed under the control of regulatory elements permitting its expression.

Said "vector" would more generally be any vehicle capable of facilitating the transfer of a gene to the endocrine cells. Preferably, it would transport the nucleic acid to cells with reduced degradation relative to the extent of degradation that would result in the absence of same. In general, the vectors useful in the invention include, but are not limited to, plasmids, phagemids, viruses, other vehicles derived from viral or bacterial sources that have been manipulated by the insertion or incorporation of the KDM1A nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus; lentivirus; cytomegalovirus; adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art. Preferably, said virus should be a defective virus. The term "defective virus" denotes a virus incapable of replicating in the target cell. Generally, the genome of the defective viruses used in the context of the present invention hence lacks at least the sequences needed for the replication of the said virus in the infected cell. These regions may be either removed (wholly or partially), or rendered non-functional, or replaced by other sequences, in particular by the recombinant nucleic acid. Preferably, the defective virus nevertheless retains the sequences of its genome which are needed for encapsulation of the viral particle.

The vector of the invention would be contained in a pharmaceutical composition. This composition would moreover contain a pharmaceutically acceptable excipient.

The term "pharmaceutically acceptable excipient" means herein an excipient that is useful in preparing a pharmaceutical composition that is generally safe, non-toxic, and desirable, and includes excipients that are acceptable for veterinary use as well as for human pharmaceutical use. Such excipients can be solid, liquid, semisolid, or, in the case of an aerosol composition, gaseous.

Any other appropriate treatment, as proposed in the literature and in particular in Ghorayeb et al³⁷, could be also administered to the patient. In particular, it is also possible to contemplate removing the diseased endocrine tissue (i.e., the endocrine tissue where the somatic invalidating mutation has occurred), e.g. by surgery, from the diagnosed subject, before cortisol excess is measurable and established.

### Primers and probes

In a particular aspect, the present application relates to primers or probes that can be used in the above-cited methods so as to detect specifically the presence of an invalidating mutation in the *KDM1A* gene or in fragments thereof, or the expression level of the *KDM1A* gene.

In this aspect, the present invention relates to the use of primers that can specifically amplify the genomic region of SEQ ID NO:1 or SEQ ID NO:2, or fragments thereof, as defined above. These primers preferably contains 18 to 30 consecutive nucleotides of SEQ ID NO:1 or SEQ ID NO:2,, or of its fragment. Preferably, they contain between 18 and 30 nucleotides (in total).

Examples of useful primers (that allow the amplification of fragments of SEQ ID NO: 1 or SEQ ID NO:2 in order to quantify them) are of SEQ ID NO:4-5. These sequences are given below and in the enclosed listing.

| | |
|---|---|
| SEQ ID NO :4 | CAGCCGATTCCACGACTCTT |
| SEQ ID NO :5 | CTCAGCAGAGCACCATGCA |

Also, the present invention relates to probes that can specifically hybridize the genomic region of SEQ ID NO:1 or SEQ ID NO:2, or fragments thereof, as defined above. In a preferred embodiment, these probes comprise at least 15, preferably at least 20, more preferably at least 30 consecutive nucleotides of SEQ ID NO:1 or SEQ ID NO:2 or fragments thereof. In a more preferred embodiment, the molecules which can be used as a probe according to the present invention have a total minimum size of 15 nucleotides, preferably of 20 nucleotides. In an even more preferred embodiment, these molecules comprise between 15 and 40 nucleotides (in total).

The probes of the invention can be carried out in diverse ways. The most general method consists in immobilizing the nucleic acid molecules extracted from the biological sample on a support (such as nitrocellulose, nylon or polystyrene), and in incubating the immobilized target nucleic acid with the probe, under well-defined conditions. After hybridization, the excess probe is eliminated and the hybrid molecules formed are detected using the appropriate method (measurement of the radioactivity, of the fluorescence or of the enzymatic activity linked to the probe).

According to another embodiment, the probe of the invention can be used as a capture probe. In this case, the probe is immobilized on a support and is used to capture, by specific hybridization, the target nucleic acid obtained from the biological sample to be tested. The target nucleic acid is then detected using a second probe, termed "detection probe", which is labeled with an easily detectable element.

The present invention relates to the use of these probes or primers for analyzing, detecting, identifying, or assaying the genomic region having the SEQ ID NO:1 or SEQ ID NO:2, or of fragments thereof, so as to identify a genetic predisposition to or to diagnose an endocrine disease in a subject in need thereof, e.g., in the methods disclosed above.

In the methods of the invention, it is also possible to use any antibody that can detect specifically the KDM1A protein of SEQ ID NO:3.

For example, it is possible to use the antibody Ab29195 (Abcam) recognizing the amino acids 50 to 150 of SEQ ID NO:3, namely : GPGAVGERTP RKKEPPRASP PGGLAEPPGS AGPQAGPTVV PGSATPMETG IAETPEGRRT SRRKRAKVEY REMDESLANL SEDEYYSEEE RNAKAEKEKK (SEQ ID NO:16).

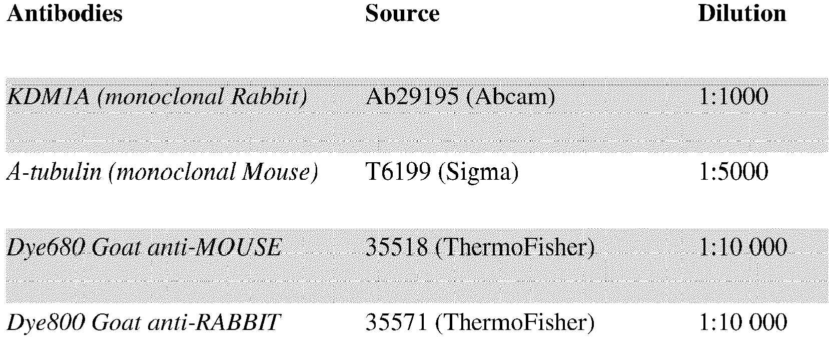

### Kit

In another aspect, the present invention refers to the use of a kit comprising at least one primer, one probe or one antibody as defined above, and its use for identifying a genetic predisposition to an endocrine disease or diagnose an endocrine disease in a subject in need thereof, e.g., in the methods disclosed above.

In a preferred embodiment, the kit of the invention contains at least two primers amplifying specifically nucleic acids having the sequence SEQ ID NO:1 or SEQ ID NO:2, or a fragment thereof, and/or at least one probe hybridizing specifically a nucleic acid having the sequence SEQ ID NO:1 or SEQ ID NO:2.

Preferably, said primers / probes have the sequence mentioned above (SEQ ID NO:4-5).

Alternatively, the kit of the invention can contain polyclonal or monoclonal antibodies which recognize specifically the KDM1A protein (SEQ ID NO:3 or the epitope of SEQ ID NO:16).

In a more preferred embodiment, the kit of the invention comprises any combination of said primers, probes and antibodies.

The present kit can also include one or more reagents, buffers, hybridization media, nucleic acids, primers, nucleotides, probes, molecular weight markers, enzymes, solid supports, databases, computer programs for calculating dispensation orders and/or disposable lab equipment, such as multi-well plates, in order to readily facilitate implementation of the present methods. Enzymes that can be included in the present kits include nucleotide polymerases and the like. Solid supports can include beads and the like whereas molecular weight markers can include conjugatable markers, for example biotin and streptavidin or the like.

In one embodiment, the present kit also contains instructions for carrying out the methods of the invention. The instructions can be provided in any intelligible form through a tangible medium, such as printed on paper, computer readable media, or the like.

### Methods for treating diabetes mellitus

Further the physiological eutopic GIP receptor expression in the pancreas (alpha, beta and delta cells of the endocrine pancreas) and other tissues may be epigenetically regulated by KDM1A, which can be pharmacologically targeted to modify insulin secretion.

It has been shown that knockdown of KDM1A in human induced pluripotent stem cells with shRNA promotes differentiation such stem cells into insulin producing cells (Yang et al. Stem Cell Research & Therapy 2020). KDM1A therefore plays an important role in beta-cell homeostasis. GIP receptor is physiologically expressed in pancreatic betacells secreting insulin.

It can thus be assumed that pharmacological inhibition of KDM1A could increase insulin secretion in patients with diabetes mellitus by epigenetic regulation of GIP receptor expression and subsequently enhancing endogenous postprandial insulin secretion.

KDM1A inhibitors could therefore potentially serve as new adjuvant treatment of diabetes mellitus, thereby increasing the GIP receptor expression in pancreatic betacells and therefore improving endogenous postprandial insulin secretion.

KDM1A antagonists are known in the art. They are for example described in Hamamoto et al, Nature Rev Cancer 2015, Karadaikos et al Cancers 2019, Fang Journal of Hematology & Oncol 2019. Any efficient KDM1A antagonist can be used, provided that it can enter into pancreatic cells.

In particular, KDM1A antagonists can be chemical compounds or blocking antibodies (or fragments thereof or aptamers) that impair KDM1A enzymatic activity in pancreatic cells.

Alternatively, siRNA or other antisense nucleic acids can be used for inhibiting the expression of the *KDM1A* gene in pancreatic cells. These inhibitors of gene expression are for example anti-sense RNA molecules and anti-sense DNA molecules, act to directly block the translation of the mRNA by binding thereto and thus preventing protein translation or increasing mRNA degradation, thus decreasing the level of the protein (e.g. the KDM1A protein), and thus its activity, in the target cell. Antisense oligonucleotides of at least about 15 bases and complementary to unique regions of the mRNA transcript sequence encoding the targeted KDM1A protein can be used.

They can be synthesized, e.g., by conventional phosphodiester techniques, and administered to the patients, by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732).

Ribozymes can also function as inhibitors of gene expression for use in the present invention. Ribozymes are enzymatic RNA molecules capable of catalysing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyse endonucleolytic cleavage of mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

The present invention therefore targets KDM1A antagonists, such as chemical or peptide compounds or KDM1A antisense nucleic acids or ribozymes, for use for treating patients suffering from diabetes mellitus in order to enhance receptivity to GIP and thus improve endogenous insulin secretion.

It also targets the use of KDM1A antagonists, such as chemical compounds or KDM1A antisense nucleic acids, for the preparation of a medicament intended to be used for treating patients suffering from diabetes mellitus.

Antisense oligonucleotides siRNAs and ribozymes of the invention may be delivered in association with a vector. In its broadest sense, a "vector" is any vehicle capable of facilitating the transfer of the antisense oligonucleotide siRNA or ribozyme nucleic acid to the pancreatic cells and preferably cells expressing the targeted KDM1A proteins. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus; lentivirus; cytomegalovirus; adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art. Preferably, said virus should be a defective virus. The term "defective virus" denotes a virus incapable of replicating in the target cell. Generally, the genome of the defective viruses used in the context of the present invention hence lacks at least the sequences needed for the replication of the said virus in the infected cell. These regions may be either removed (wholly or partially), or rendered non-functional, or replaced by other sequences, in particular by the recombinant nucleic acid. Preferably, the defective virus nevertheless retains the sequences of its genome which are needed for encapsulation of the viral particle.

The advantage of the present invention is to extend the use of existing KDM1A inhibitors and those which be developed to a completely new field of epigenetic regulation of tissue-specific expression of GPCRs, including, but not limited to, eutopic expression of GIP receptor in the pancreas.

### EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### 1. METHODS

### Study oversight

Patients with PBMAH gave written informed consent for genetic analyses. The study was approved by all local Ethics Committees. The tissue samples from patients with PBMAH without ectopic GIP receptor expression were obtained from the Centre de Ressources Biologiques Paris-Saclay (BRIF: BB-0033-00089, Bicetre Hospital, AP-HP).

### Patients

Adrenal samples from patients who had undergone adrenalectomy for GIP-dependent Cushing's syndrome were included in the study. This international tissue collection included adrenal samples from six endocrine centers in France, Belgium and Canada. GIP-dependent Cushing's syndrome was diagnosed according to standard criteria showing hypercortisolism¹ in combination with low morning (fasting) plasma cortisol and adrenocorticotropin concentrations with a meal-induced (or other tests including GIP infusion) increase of plasma cortisol concentration. Control samples of PBMAH derived from patients who had undergone adrenalectomy at Bicêtre hospital for overt or subclinical Cushing's syndrome without evidence of food-dependent cortisol production, were also included.

### Genotyping and gene copy number analysis

DNA was extracted from adrenal samples using the phenol-chloroform-isoamyl alcohol extraction method. DNA was extracted from Formalin-Fixed Paraffin-Embedded (FFPE) sample using NucleoSpin DNA FFPE XS kit (Macherey-Nagel) or from blood leucocytes using either an automated QIAsymphony platform (Qiagen) or the Flexigene DNA Kit (Qiagen).

**Whole genome sequencing.** PCR free libraries were obtained with NEBNext^{®} Ultra^{™} II DNA Library Prep Kit according to the supplier's recommendations. DNA PCR free library was sequenced on paired-end 150 bp run on the Illumina NovaSeq. Sequence reads were mapped to the Human genome hg38 by using the Burrows-Wheeler Aligner tool. A mean coverage of 49X was obtained. Variant calling and annotations were made using GATK haplotype caller GVCF tool, V 3.7 for constitutional DNA and MuTect tool, V 2.0 for somatic DNA (both from Broad institute, Cambridge, MA, USA). Variants with a minor allele frequency greater than 2% in gnomAD or 1000 genomes project database were excluded. The whole-genome-sequencing data was further filtered to keep protein-damaging variants (nonsense, missense, frameshift, indel and splice variants).

**Whole exome sequencing** was performed as previously described⁶. All coding exons were captured using the SureSelect XT Human All Exon (Agilent Technologies). Sequencing was performed on an Illumina HiSeq 4000 with paired-end 100-bp reads. Read alignment (aligned to Human genome hg19), variant calling, and annotation were done with a pipeline based on Burrows-Wheeler Aligner, SAMtools, Annovar, and custom annotation scripts. A mean coverage of 100X was obtained. Variants with minor allele frequency greater than 1% in either the 1000 genomes project database, the 6500 NHLBI EVS (http://evs.gs.washington.edu/ EVS), gnomAD and seen in more than 30 samples from our in-house database (containing approximately 2000 samples) were excluded. The whole-exome-sequencing data were further filtered to keep protein-damaging variants.

**Targeted next generation sequencing** was further used to sequence with high resolution somatic DNA from all patients and germline DNA when available with a panel of genes involved in adrenal diseases, including ARMC5, PRKACA, PRKACB, PRKAR1A, GNAS, MEN1, and FH. The panel also included members of the lysine demethylase family involved in human tumorigenesis KDM1A, KDM3A, JMJD2A, JMJD2B, JMJD3, KDM6A and KDM5B7. Whole genome libraries were prepared using NEBNext@ Ultra^{™} II FS DNA Library Prep Kit for Illumina (NEB Inc.) robotized on a Biomek Span 8 workstation (Beckman). Enrichment was processed using SureSelect XT custom kit (Agilent) robotized on a Biomek 4000 workstation (Beckman). Paired-end 2×150 bp sequencing was performed by batch of 23 patients on a Miseq^{®} IlluminaTM sequencer. Read alignment (aligned to Human genome hg19), variant calling, and annotation were done with Annovar and SNPeff 4.0. A mean coverage of 286X was obtained. To investigate genomic copy number alterations (CNA) based on Targeted Next Generation Sequencing data, the in-house Python (V2.7) script was used to compare the depth of coverage of samples from patients versus control samples.

**Oligonucleotide based array-comparative genomic hybridization analysis** (array-CGH) was used to analyze genomic imbalances using 180K or 400K oligonucleotide arrays (Agilent Technologies). DNA from adrenal samples was compared to sexmatched blood donor DNA. Hybridization was performed according to the manufacturer's protocol. The slides were scanned on an Agilent Microarray Scanner. Images processing and data analysis was performed with CytoGenomics software 4.0.3.12 (Agilent Technologies). ADM2 algorithm was used for statistical analysis. Copy number alterations were considered significant if they could be defined by 3 or more oligonucleotides spanning at least 15Kb and 35Kb (for 180K and 400K arrays, respectively), and were not identified in the Database of Genomic Variants (http://projects.tcag.ca/cgi-bin/variation/gbrowse/hg19). The Genome Browser used to analyze genes content was hg19, Build37 (http://genome.ucsc.edu/).

*Cell culture of human adrenocortical H295R cells.*

H295R cells were cultivated in DMEM/Ham's F-12 Medium supplemented with 2 mM Glutamine, 100 IU/mL Penicillin, 100 µg/mL Streptomycin, 10% Fetal Bovine Serum, 20 mM HEPES, and a mixture of Insulin (1µM), Transferrin (5.8 mg/ml) and Selenium (60nM) .

### Inhibition of KDM1A

Cells were incubated up to 10 days with 0.5 µM GSK-LSD1 an irreversible inhibitor of KDM1A (Sigma-Aldrich).

For genetic inhibition, the following siRNAs were used.

| | | |
|---|---|---|
| si-RNA J-009223-05 | GGAAGUUGUCAUUCAGUUA | SEQ ID NO: 12 |
| si-RNA J-009223-06 | CCACCGAGUUCACAGUUAU | SEQ ID NO: 13 |
| si-RNA J-009223-07 | CAUAAGUGACGAUGUGAUU | SEQ ID NO: 14 |
| si-RNA J-009223-08 | CUAUAAAGCUCCAAUACUG | SEQ ID NO: 15 |

Transfection of siRNAs protocol was performed as follow. Briefly, H295R cells were cultivated in serum free Optimem medium (Life Technologies) for 2 hours, then incubated for 6h with Lipofectamine RNAiMAX (2µL/well) and 100nM siRNA targeting KDM1A or Scrambled siRNA (Horizon). Two iterative transfections were performed at 1 and 3 days after seeding. Expression level of the studied gene was determined using reverse transcription quantitative polymerase chain reaction (RT-qPCR).

### Measurement of gene levels

RNA extraction from cell cultures and tissue protocol and RT-qPCR protocol were as follows: DNA was extracted from frozen adrenal samples using the phenol-chloroform-isoamyl alcohol extraction method. DNA was extracted from Formalin-Fixed Paraffin-Embedded (FFPE) sample using NucleoSpin DNA FFPE XS kit (Macherey-Nagel) or from blood leucocytes using either an automated QIAsymphony platform (Qiagen) or the Flexigene DNA Kit (Qiagen). Total RNA was extracted from tissues or cells using TRIzol reagent according to the manufacturer's instructions. After DNAse I treatment (New England Biolabs), 1 µg of total RNA was reverse transcribed using the High-Capacity cDNA reverse transcription kit (Life Technologies). Samples were analyzed by RT-qPCR using the Power SYBR^{®} Green PCR Master Mix (Life Technologies) or the TaqMan Gene Expression Assays (Life Technologies, ref. 4331182 and 4448489) and were run on a QS6 Real-Time PCR System (Life Technologies). Standard curves were generated by use of serial dilutions of linearized pGEMT-easy plasmid (Promega), in which the amplicon was previously cloned and sequenced. Relative expression of the gene of interest was expressed as a ratio of attomoles to attomoles of housekeeper genes.

Primers and Taqman probes are indicated below:

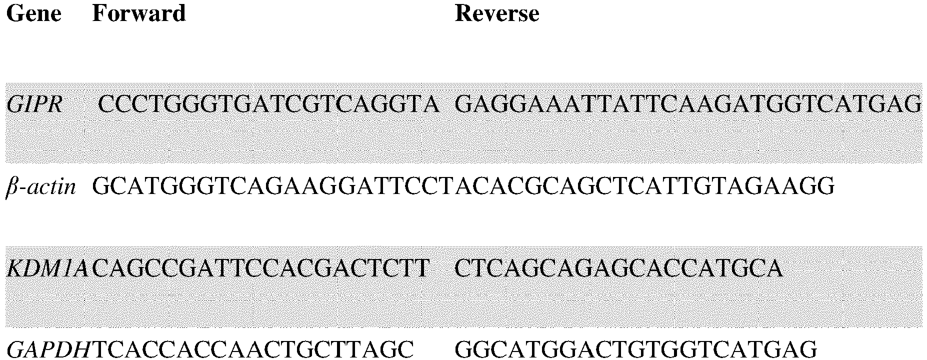

These primers are disclosed as SEQ ID NO:4-11.

### Measurement of protein levels

Adrenal protein extracts were obtained with a TissueLyser (Qiagen). Details of Western Blot analysis are as follows: adrenal protein extracts were obtained with a Tissuelyser (Qiagen). Ten micrograms of proteins were subjected to SDS-PAGE and processed for detection of KDM1A protein together with α-tubulin protein for loading normalization. Migration was performed on a 7% acrylamide/bis-acrylamide resolving gel and a 4% acrylamide/bis-acrylamide stacking gel during 10 min at 80 V and 1 h at 150 V. Transfer was performed on a nitrocellulose membrane. Blocking and dilution of antibodies were performed using 0.1% TBS-T buffer with 3% milk. The fluorescence signal intensity was determined with an Odyssey^{®} (Li-Cor). Data were analyzed with Image Studio 1.1 software (Li-COR). Antibody sources and dilutions are given below.

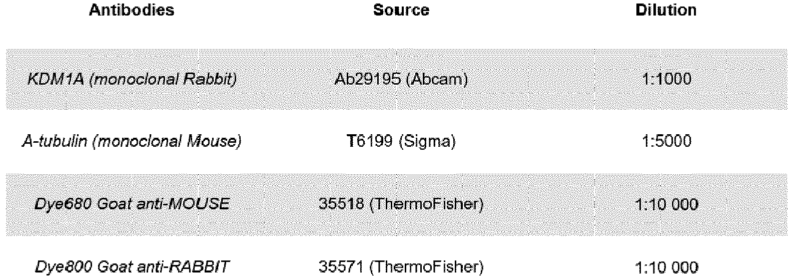

### Immunocytofluorescence

Cells were grown on a 24 well plates containing glass coverslips (14-mm diameter). After 24h, siRNA targeting KDM1A or Scramble siRNA (100nM) were transfected as described above. Briefly, cells were fixed with 4% paraformaldehyde and permeabilized 10 min with a 0.2% solution of Triton X100 diluted in PBS. Cells were then incubated overnight at 4°C with a monoclonal rabbit anti-GIPR antibody (US biological) following by a 60 min incubation with an anti-rabbit alexa 555-coupled secondary antibody (Life Technologie). Counter staining of nuclei was performed with 0.5µg/mL DAPI (4',6'-diamidino-2-phenylindole) and coverslips were mounted on slides with ProLong Gold mounting medium (Life Technologie).

### Fluorescence deconvolution microscopy

Cells were observed and acquired with an automated upright BX61 microscope (Olympus) at 100X objective lens (1.4 NA) using a Mono Q Imaging Retiga 2000R Fast 1394 camera (Q Imaging Inc.). Deconvolution process was performed with Image Pro Plus AMS (Media Cybernetics Inc): a z-series of focal planes was digitally imaged and deconvolved with the 3D blind iterative algorithm (Image Pro Plus AMS) to generate high-resolution images.

### Automated quantification of GOPR fluorescence by high throughput microscopy

Sequential images (20X-0.40 NA lens) were acquired for each channel with the automated ArrayScan VTI imaging and analyzing platform (Thermo Fisher Scientific). The TargetActivation Algorithm (v.4) was used to detect and quantify the GIPR fluorescence as following. DAPI fluorescence was used to find the focus and was sequentially acquired with GIPR fluorescence in order to give separate image files. The DAPI staining (channel 1) identifying the nuclear region was used to define a binary nuclear mask. Nuclei clusters, mitotic cells, and apoptotic cells were gated out from the total cell population (total primary object) by using several progressive morphological filters. In order to quantify the amount of GIPR fluorescence (channel 2) present in the cytoplasm of the primary selected object, this nuclear mask was dilated to cover the cytoplasmic region. Subtraction of the nuclear from the dilated mask created a binary cytoplasmic new mask covering the cytoplasmic region. An automatic cut-off threshold was used to specifically select the cells expressing GIPR (secondary selected object, i.e. transfected cells). The cytoplasmic mask was lastly used to detect, select and quantify the average fluorescence of GIPR in the final population of transfected cells (n>40,000). The images and masks were systematically visually inspected for accuracy.

### Statistical analysis

Data are expressed as mean ± SEM. Differences between groups were analyzed by nonparametric

Mann-Whitney test, parametric unpaired t-test or Kruskal-Wallis test, followed by Dunn's post-test (Prism 9, GraphPad Software). P values less than 0.05 were considered statistically significant.

### 2. RESULTS

### Clinical presentation

Twelve patients with overt GIP-dependent Cushing's syndrome with PBMAH treated with unilateral or bilateral adrenalectomy were studied (table 1).

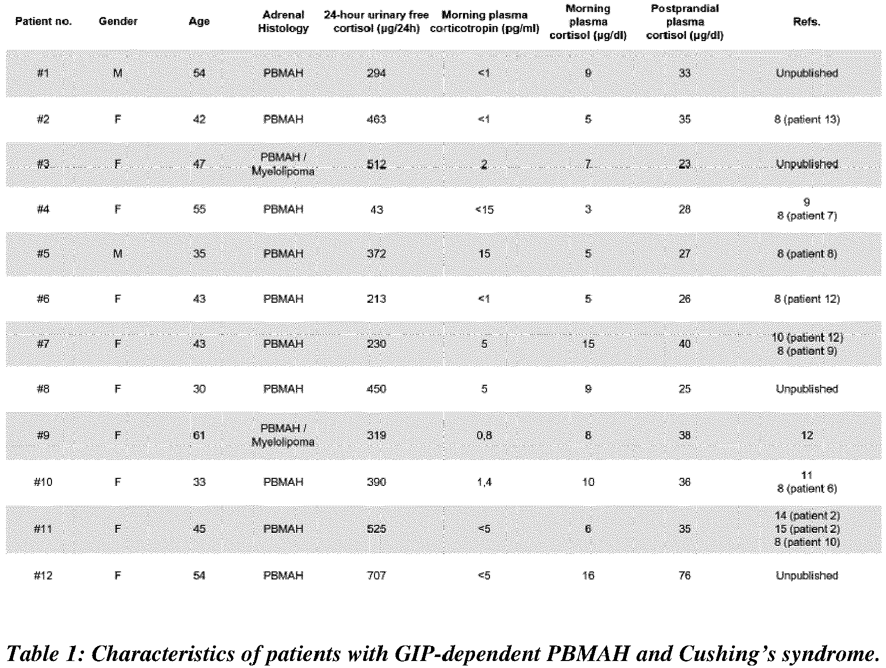

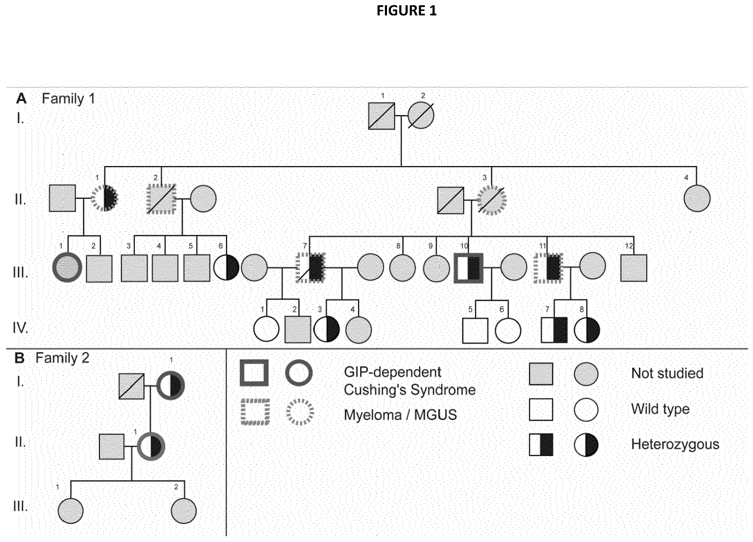

Three patients belonged to two unrelated families with GIP-dependent Cushing's syndrome (figure 1), and 9 were apparently sporadic cases. Two members of the family 1 (index case: patient #1 and his first-degree female cousin, not studied) presented with GIP-dependent Cushing's syndrome. Family medical history also included several cases of multiple myeloma, monoclonal gammopathy of undermined significance (MGUS), rectal cancer at 35 years-old and a bronchial neuroendocrine tumor diagnosed at 80 years-old (figure 1). Two members of family 2 (patients #2 and #3) presented with GIP-dependent Cushing's syndrome (figure 1). Patient #3 presented with a myelolipoma intricated with the hyperplastic left adrenal tissue; no other neoplasia was reported in her family.

Amongst the nine apparently sporadic cases, seven were previously published ⁸⁻¹⁵. Patient #4 had a daughter presenting a craniopharyngioma in infancy and a first-degree female cousin who underwent unilateral adrenalectomy for an adrenal mass (no further details available). Patient #9 had several foci of myelolipoma within both PBMAH glands ¹². Patient #10 had a breast cancer at 42 years-old, patient #11 had a mother and a sister presenting with breast cancer. Patient #12 had a brother with Hodgkin lymphoma.

Sixteen patients with PBMAH who underwent unilateral or bilateral adrenalectomy for sub-clinical or clinical adrenocorticotropin-independent Cushing's syndrome without evidence of food-dependent cortisol production were included as controls. Amongst them two were members of an ARMC5-mutated family and presented with β-adrenergic/vasopressin responsive Cushing's syndrome with no proven food-dependent cortisol secretion ^{16,17}. Other PBMAH controls were apparently sporadic cases.

### Loss of heterozygosity in chromosome 1p in adrenal lesions

Array-CGH and targeted NGS were used to map DNA copy number alterations in all twelve PBMAH samples derived from patients with GIP-dependent Cushing's syndrome. Low copy number alterations were detected in these samples. However, a recurrent deletion of the short arm of chromosome 1 was identified, present in all but two samples. Although array-CGH did not detect chromosome 1 deletion in adrenal samples from patients #9 and #12, targeted NGS identified microdeletions in 1p36.12 in one of these patients (patient #12).

### Germline inactivating mutations of KDM1A

Whole Genome Sequencing from both adrenal lesions and germline DNA derived from the index patient of family 1 (figure 1A) identified mutations affecting the coding sequence of 69 candidate genes common in all 3 samples. Crossing the Whole Genome Sequencing analysis with the recurrent 1p deletion pointed to three candidate genes. In particular, a heterozygous germline frameshift mutation was identified, leading to a premature stop codon in exon 16 of *KDM1A (NM_001009999.3),* located in 1p36.12 (figure 2). The mutation was not reported in gnomAD nor in the 1000 genomes project databases. In parallel, whole exome sequencing was performed on germline DNA from the apparently sporadic case #9 and revealed a heterozygous germline nonsense mutation in exon 6 of *KDM1A* (figure 2).

Systematic analysis using targeted next generation sequencing of DNA from the remaining patients with PBMAH GIP-dependent Cushing's syndrome identified *KDM1A* mutations in all adrenal hyperplasia samples, including 5 frameshift mutations, 5 nonsense mutations and one splicing mutation (figure 2). All mutations were considered pathogenic according to the ACMG classification and were not reported in gnomAD nor the 1000 genomes project databases. The presence of these mutations was confirmed in germline DNA of ten patients (figure 2). Finally, no pathogenic mutations in *ARMC5* gene or other known genes involved in adrenal tumors or in other histone demethylases genes were identified in these patients.

The germline KDM1A mutations detected in the patients suffering from GIP-dependet Cushing's syndrome and PBMAH are displayed on Table 2.

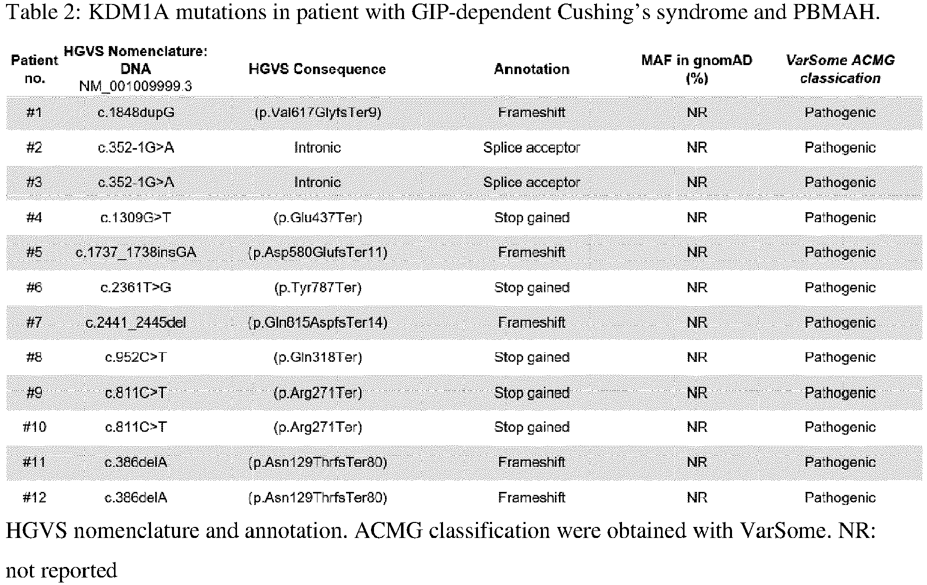

Targeted exome sequencing of control samples did not find pathogenic variants in *KDM1A,* neither copy number alteration in 1p36.12 in any of these samples (not shown). This analysis identified *ARMC5* mutations in 5 control samples (31%). Western blot analysis demonstrated loss of expression of KDM1A protein in adrenal lesion samples derived from patient with GIP-dependent Cushing's syndrome (not shown), whereas KDM1A protein was detected in controls. Thus, germline mutations *in KDM1A* in combination with 1p loss of heterozygosity results in a functional loss of KDM1A in the adrenal lesions of affected patients.

### Loss-of-function of KDM1A results in GIP-receptor expression

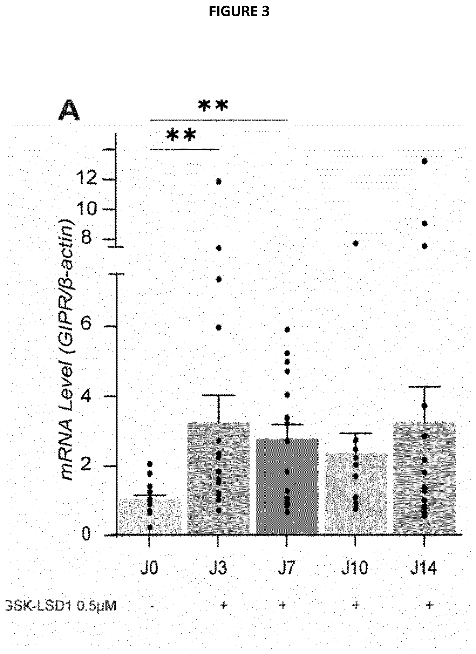

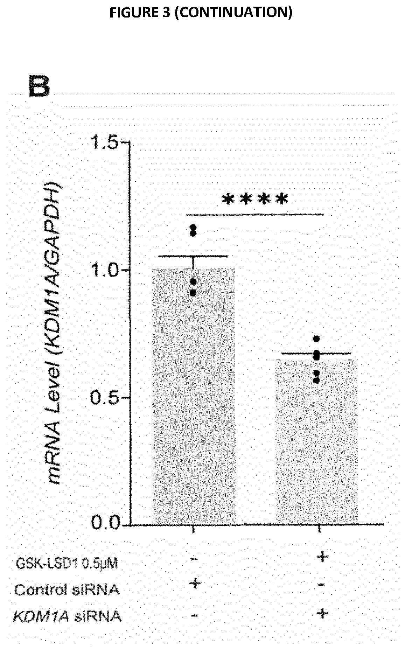

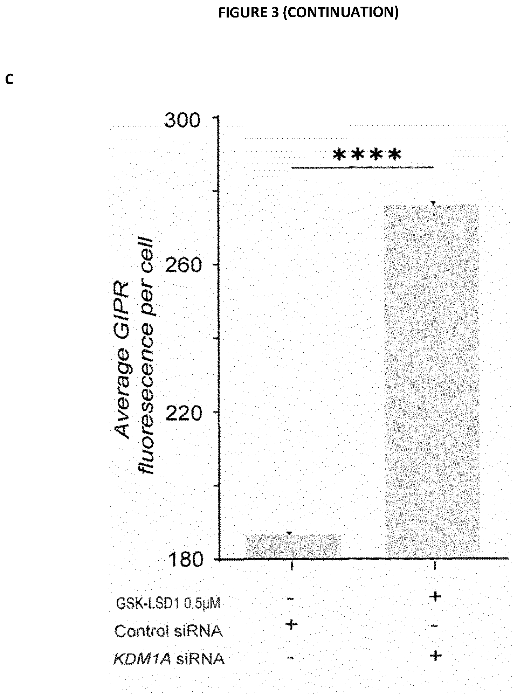

*In vitro* studies in human adrenocortical H295R cells confirmed the functional consequence of the loss of KDM1A on GIPR expression (figure 3). Treatment with GSK-LSD1, a selective and non-reversible inhibitor of KDM1A resulted in increased GIPR expression in H295R cells, quantified by RT-qPCR. To further investigate the impact of KDM1A on GIPR expression at the protein level, GIPR expression was analyzed by automated High-throughput Microscopy after KDM1A silencing (siRNA) and pharmacological inhibition with GSK-LSD1. Both inhibitions induced a significant increased expression of GIPR protein in H295R cells compared to untreated cells (number of analyzed cells > 40 000, p < 0.001).

### 3. DISCUSSION

Germline inactivating mutations were detected in the lysine demethylase *KDM1A* in two families with PBMAH and GIP-dependent Cushing's syndrome. Such pathogenic germline *KDM1A* genetic alterations were identified in all patients who presented with sporadic GIP-dependent Cushing's syndrome with PBMAH, suggesting a common genetic mechanism of this disease. This was further supported by the loss of the second *KDM1A* locus in all adrenal lesions of affected patients. All reported *KDM1A* mutations were truncating or frameshift mutations not reported in gnomAD nor in the 1000 genomes project databases. It is noteworthy that *KDM1A* has a pLI score equal to 1, reflecting the very low tolerance of the gene to protein truncating variants ¹⁸. Finally, none of the control subjects harbored *KDM1A* germline and somatic alterations.

It is herein proposed a two-hits scenario of *KDM1A* inactivation, consistent with the tumor suppressor gene model of tumorigenesis ¹⁹. This pathogenesis is similar to adrenal tumor formation observed in type 1 multiple endocrine neoplasia^{20,21} and in *ARMC5* mutation-related PBMAH development². No *ARMC5* mutations were detected in any of these patients with GIP-dependent PBMAH or in the literature²²; similarly, none of the *ARMC5*-mutation positive control cases harbored *KDM1A* mutations. Thus, genetic alterations in *ARMC5* and *KDM1A* genes appear to be mutually exclusive driver events responsible for molecular pathogenic mechanisms of PBMAH. Furthermore, as previously reported⁸, GIPR expressing adrenal adenoma development may result from somatic 19q13 microduplications with chromosome rearrangements and have different molecular pathogenesis. These chromosome rearrangements, generated a novel genomic environment by juxtaposing the *GIPR* gene with cis-acting regulatory sequences, permitting its adrenal expression⁸.

Similarly to *ARMC5* mutation-related adrenal disease ², step-wise inactivation of *KDM1A* is associated with insidious development of adrenal masses, with a median age of PBMAH and GIP-dependent Cushing's syndrome diagnosis at the age of 44 years. Along this line, the screening of four clinically unaffected *KDM1A* mutation carriers in family 1 did not detect any biochemical abnormalities at younger ages (not shown).

*KDM1A* encodes for a histone lysine demethylase, belonging to a larger family of such proteins⁷. Histone tails are subjected to covalent modifications that affect chromatin structure and the recruitment of regulatory factors consequently modifying transcription. Methylation of lysine residues can be associated with either activation or repression of transcription²³. KDM1A represses transcription by demethylating histone H3 on lysin 4 (H3K4me) a histone mark usually linked to active gene transcription ^{23,24}. In addition, KDM1A has also been shown to affect methylation of non-histone proteins involved in tumorigenesis such as p53, RB1 and STAT3 ⁷. Both mechanisms can be of importance in the pathogenesis of GIP-dependent Cushing's syndrome with PBMAH. Persistent histone methylation secondary to loss of KDM1A function can result in aberrant transcriptional activation, and absence of KDM1A interaction with oncogenic proteins that can lead to cell cycle dysregulation and consequently adrenal tumorigenesis. *In vitro* pharmacologic inhibition or silencing of KDM1A with siRNAs resulted in an increase in GIPR transcripts and protein in human adrenocortical cells. Targeted exome sequencing did not detect any additional molecular events in genes known to be involved in adrenal tumorigenesis. Thus, loss of KDM1A function seems sufficient to induce aberrant GIPR expression in adrenal cells. In two cases (patients #3 and #9), the adrenal hyperplasia included foci of lipomatous tissue with areas of hematopoiesis, with a potential role of KDM1A inactivation in the development of adrenal lesions with complex histological architecture. Interestingly KDM1A has been reported to be a key epigenetic regulator of hematopoietic differentiation and is involved in the hematopoietic commitment of hemangioblasts²⁵.

Wei et al. reported that germline *KDM1A* mutations are a rare cause of familial or early-onset multiple myeloma²⁶. Interestingly, several members of family 1 carrying *KDM1A* mutations were affected by multiple myeloma or MGUS. This observation confirms the role of *KDM1A* as germline multiple myeloma predisposition gene. In addition, somatic *KDM1A* alterations have been described in lung, colorectal or breast cancer and in acute myeloid leukemia ²⁷⁻³¹. Occurrence of such cancers in several patients in the present study, or in their kindreds points to a more general implication of *KDM1A* disruption in tumor development. GIPR expression has also been reported in neuroendocrine tumors and somatotroph pituitary adenomas and linked with DNA hypermethylation ^{32,33}. As DNA methylation and histone methylation are inextricably interlaced³⁴, *KDM1A* and other demethylase genes appear as new candidates potentially involved in endocrine tumorigenesis.

In conclusion, it is demonstrated here that germline inactivating *KDM1A* mutations can act as a genetic predisposition to PBMAH with GIP-dependent Cushing's syndrome. Somatic loss of heterozygosity of the KDM1A locus represents the prerequisite for the adrenal disease development. Hence, PBMAH with aberrant GIPR expression is a genetic disease. Thus, identification of GIP-dependent PBMAH with Cushing's syndrome should lead to biochemical and genetic screening of kindreds.

### FIGURE LEGENDS

**Figure 1** describes the family pedigrees of patients with familial PBMAH and GIP-dependent Cushing's syndrome that have been studied in the examples below.
   Panel A shows the family pedigree of family 1, with two members affected by GIP-dependent Cushing's syndrome with primary bilateral macronodular adrenal hyperplasia (PBMAH, indicated in plain lines) and 5 members affected by multiple myeloma or monoclonal gammopathy of undetermined significance (indicated in doted lines). Patient III.10 is patient #1 (Table 1). Panel B shows the family pedigree of family 2, with two members affected by GIP-dependent Cushing's Syndrome with PBMAH (indicated in plain lines). Patient II.1 and I.1 are patient #2 and #3 (cf. Table 1). Circles represent female and squares represent male family members. Half shaded symbols represent members carrying a heterozygous KDM1A mutation, white symbols indicate members with wild type KDM1A gene, grey symbols indicate members not studied. Crossed out symbols represent deceased individuals.
**Figure 2** shows the germline (A) and sporadic adrenal alterations (B) in KDM1A in patients with GIP-dependent PBMAH and Cushing's syndrome. Further, loss-of heterozygosity in chromosome 1p, harboring the *KDM1A* locus, identified by array-CGH are shown (line below). In patients #9 and #12, loss of heterozygosity in 1p was not detected by array-CGH, but NGS uncovered somatic microdeletion of the *KDM1A* locus in patient #12.
**Figure 3** discloses the results of *in vitro* studies of functional KDM1A inhibition in adrenocortical H295R cells. Panel A shows the effect of pharmacological inhibition of KDM1A by GSK-LSD1 at 0.5µM in H295R cells. GIPR messenger RNA level was quantified by RT-qPCR. Functional inhibition of KDM1A results in significant increase of GIPR expression at three- and seven-days time points (p=0.0069 and p=0.0082). Panel B shows RT-qPCR quantification of KDM1A expression in H295R cells after seven days of treatment with 0.5µM GSK-LSD1 and two iterative transfections with KDM1A siRNA. Significant decrease is observed after siRNA transfection (p<0.0001). The C panel shows that KDM1A siRNA transfection and concomitant pharmacological inhibition for seven days resulted in significant GIPR expression, as observed by immunofluorescence. Induction of GIPR protein expression in the cytoplasm of H295R cells was quantified using automated High-throughput Microscopy. Transfected and treated H295R cells presented an increase in protein expression (p<0.0001, number of analyzed cells>40 000).

### BIBLIOGRAPHIC REFERENCES

1. Lacroix A, Feelders RA, Stratakis CA, Nieman LK. Cushing's syndrome. The Lancet 2015;386(9996):913-27.
2. Assié G, Libé R, Espiard S, et al. ARMC5 Mutations in Macronodular Adrenal Hyperplasia with Cushing's Syndrome. N Engl J Med 2013;369(22):2105-14.
3. Kamenicky P, Lacroix A. Mechanism of ectopic hormone receptors in adrenal tumors and hyperplasia. Curr Opin Endocr Metab Res 2019;8:206-12.
4. Lacroix A, Bolté E, Tremblay J, Dupré J, Poitras P, Fournier H, Garon J, Garrel D, Bayard F, Taillefer R, et al. Gastric inhibitory polypeptide-dependent cortisol hypersecretion--a new cause of Cushing's syndrome. N Engl J Med. 1992 Oct 1;327(14):974-80.
5. Reznik Y, Allali-Zerah V, Chayvialle JA, Leroyer R, Leymarie P, Travert G, Lebrethon MC, Budi I, Balliere AM, Mahoudeau J. Food-dependent Cushing's syndrome mediated by aberrant adrenal sensitivity to gastric inhibitory polypeptide. N Engl J Med. 1992 Oct 1;327(14):981-6.
6. Tetreault M, Fahiminiya S, Antonicka H, Mitchell GA, Geraghty MT, Lines M, Boycott KM, Shoubridge EA, Mitchell JJ; Care4Rare Canada Consortium, Michaud JL, Majewski J. Whole-exome sequencing identifies novel ECHS1 mutations in Leigh syndrome. Hum Genet. 2015 Sep;134(9):981-91.
7. Hamamoto R, Saloura V, Nakamura Y. Critical roles of non-histone protein lysine methylation in human tumorigenesis. Nat Rev Cancer 2015;15(2):110-24.
8. Lecoq AL, Stratakis CA, Viengchareun S, Chaligné R, Tosca L, Deméocq V, Hage M, Berthon A, Faucz FR, Hanna P, Boyer HG, Servant N, Salenave S, Tachdjian G, Adam C, Benhamo V, Clauser E, Guiochon-Mantel A, Young J, Lombès M, Bourdeau I, Maiter D, Tabarin A, Bertherat J, Lefebvre H, de Herder W, Louiset E, Lacroix A, Chanson P, Bouligand J, Kamenicky P. Adrenal GIPR expression and chromosome 19q13 microduplications in GIP-dependent Cushing's syndrome. JCI Insight. 2017 Sep 21;2(18):e92184.
9. Preumont V, Mermejo LM, Damoiseaux P, Lacroix A, Maiter D. Transient Efficacy of Octreotide and Pasireotide (SOM230) Treatment in GIP-dependent Cushing's Syndrome. Horm Metab Res 2011;43(04):287-91.
10. Doppman JL, Chrousos GP, Papanicolaou DA, Stratakis CA, Alexander HR, Nieman LK. Adrenocorticotropin-independent Macronodular Adrenal Hyperplasia: An Uncommon Cause of Primary Adrenal Hypercortisolism. Radiology 2000;216(3):797-802.
11. N'Diaye N, Hamet P, Tremblay J, Boutin J-M, Gaboury L, Lacroix A. Asynchronous Development of Bilateral Nodular Adrenal Hyperplasia in Gastric Inhibitory Polypeptide- Dependent Cushing's Syndrome. J Clin Endocrinol Metab. 1999 Aug;84(8):2616-22.
12. Larose S, Bondaz L, Mermejo LM, et al. Coexistence of Myelolipoma and Primary Bilateral Macronodular Adrenal Hyperplasia With GIP-Dependent Cushing's Syndrome. Front Endocrinol 2019;10:618.
13. Lampron A, Bourdeau I, Hamet P, Tremblay J, Lacroix A. Whole Genome Expression Profiling of Glucose-Dependent Insulinotropic Peptide (GIP)- and Adrenocorticotropin-Dependent Adrenal Hyperplasias Reveals Novel Targets for the Study of GIP-Dependent Cushing's Syndrome. J Clin Endocrinol Metab. 2006 Sep;91(9):3611-8.
14. Louiset E, Contesse V, Groussin L, et al. Expression of Serotonin 7 Receptor and Coupling of Ectopic Receptors to Protein Kinase A and Ionic Currents in Adrenocorticotropin-Independent Macronodular Adrenal Hyperplasia Causing Cushing's Syndrome. J Clin Endocrinol Metab 2006;91(11):4578-86.
15. Bertherat J, Contesse V, Louiset E, et al. In Vivo and in Vitro Screening for Illegitimate Receptors in Adrenocorticotropin-Independent Macronodular Adrenal Hyperplasia Causing Cushing's Syndrome: Identification of Two Cases of Gonadotropin/Gastric Inhibitory Polypeptide-Dependent Hypercortisolism. J Clin Endocrinol Metab 2005;90(3):1302-10.
16. Lacroix A, Tremblay J, Rousseau G, Bouvier M, Hamet P. Propranolol Therapy for Ectopic β-Adrenergic Receptors in Adrenal Cushing's Syndrome. N Engl J Med 1997;337(20): 1429-34.
17. Bourdeau I, Oble S, Magne F, Levesque I, Caceres-Gorriti KY, Nolet S, Awadalla P, Tremblay J, Hamet P, Fragoso MC, Lacroix A. ARMC5 mutations in a large French-Canadian family with cortisol-secreting β-adrenergic/vasopressin responsive bilateral macronodular adrenal hyperplasia. Eur J Endocrinol. 2016 Jan;174(1):85-96.
18. Exome Aggregation Consortium, Lek M, Karczewski KJ, et al. Analysis of protein-coding genetic variation in 60,706 humans. Nature 2016;536(7616):285-91.
19. Knudson AG. Antioncogenes and human cancer. Proc Natl Acad Sci USA. 1993 Dec 1;90(23):10914-21.
20. Larsson C, Skogseid B, Oberg K, Nakamura Y, Nordenskjöld M. Multiple endocrine neoplasia type 1 gene maps to chromosome 11 and is lost in insulinoma. Nature 1988;332(6159):85-7.
21. Chandrasekharappa SC, Guru SC, Manickam P, et al. Positional Cloning of the Gene for Multiple Endocrine Neoplasia-Type 1. Science 1997;276(5311):404-7.
22. Espiard S, Drougat L, Libé R, et al. ARMC5 Mutations in a Large Cohort of Primary Macronodular Adrenal Hyperplasia: Clinical and Functional Consequences. J Clin Endocrinol Metab 2015;100(6):E926-35.
23. Shi Y, Lan F, Matson C, et al. Histone Demethylation Mediated by the Nuclear Amine Oxidase Homolog LSD1. Cell 2004;119(7):941-53.
24. Liang G, Lin JCY, Wei V, et al. Distinct localization of histone H3 acetylation and H3-K4 methylation to the transcription start sites in the human genome. Proc Natl Acad Sci 2004;101(19):7357-62.
25. Takeuchi M, Fuse Y, Watanabe M, et al. LSD1/KDM1A promotes hematopoietic commitment of hemangioblasts through downregulation of Etv2. Proc Natl Acad Sci 2015; 112(45):13922-7.
26. Wei X, Calvo-Vidal MN, Chen S, et al. Germline Lysine-Specific Demethylase 1 (LSD1/KDM1A) Mutations Confer Susceptibility to Multiple Myeloma. Cancer Res 2018;78(10):2747-59.
27. Takagi S, Ishikawa Y, Mizutani A, et al. LSD1 Inhibitor T-3775440 Inhibits SCLC Cell Proliferation by Disrupting LSD1 Interactions with SNAG Domain Proteins INSM1 and GFI1B. Cancer Res. 2017 Sep 1;77(17):4652-4662.
28. Jin Y, Ma D, Gramyk T, et al. Kdmla promotes SCLC progression by transcriptionally silencing the tumor suppressor Rest. Biochem Biophys Res Commun 2019;515(1):214-21.
29. Mohammad HP, Smitheman KN, Kamat CD, et al. A DNA Hypomethylation Signature Predicts Antitumor Activity of LSD1 Inhibitors in SCLC. Cancer Cell 2015;28(1):57-69.
30. Zhang Y, Wu T, Wang Y, et al. The R251Q mutation of LSD1 promotes invasion and migration of luminal breast cancer cells. Int J Biol Macromol 2020;164:4000-9.
31. Ramirez-Ramirez R, Gutierrez-Angulo M, Peregrina-Sandoval J, et al. Somatic deletion of KDM1A/LSD1 gene is associated to advanced colorectal cancer stages. J Clin Pathol 2020;73(2):107-11.
32. Karpathakis A, Dibra H, Pipinikas C, et al. Prognostic Impact of Novel Molecular Subtypes of Small Intestinal Neuroendocrine Tumor. Clin Cancer Res 2016;22(1):250-8.
33. Hage M, Chaligné R, Viengchareun S, et al. Hypermethylator Phenotype and Ectopic GIP Receptor in GNAS Mutation-Negative Somatotropinomas. J Clin Endocrinol Metab 2019; 104(5): 1777-87.
34. Wang J, Hevi S, Kurash JK, et al. The lysine demethylase LSD1 (KDM1) is required for maintenance of global DNA methylation. Nat Genet 2009;41(1):125-9.
35. Clayton RN, Jones PW, Reulen RC, Stewart PM, Hassan-Smith ZK, Ntali G, et al. Mortality in patients with Cushing's disease more than 10 years after remission: A multicentre, multinational, retrospective cohort study. Lancet Diabetes Endocrinol. 2016 Jul;4(7):569-76.
36. Drougat L, Espiard S, Bertherat J. Genetics of primary bilateral macronodular adrenal hyperplasia: a model for early diagnosis of Cushing's syndrome ? J.Eur J Endocrinol. 2015 Oct;173(4):M121-31.
37. Nada El Ghorayeb, Isabelle Bourdeau, André Lacroix, Multiple aberrant hormone receptors in Cushing's syndrome, Eur J Endocrinol. 2015 Oct;173(4):M45-60.

## Claims

1. An *in vitro* method for identifying a genetic predisposition to an endocrine disease in a subject in need thereof, said method comprising the step of analyzing a biological sample from said subject so as to detect the presence of a germline invalidating mutation in the *KDM1A* gene and/or an abnormal expression or activity of the KDM1A protein in said sample.

2. The *in vitro* method of claim 1, comprising the steps of :
(i) detecting the presence of a germline invalidating mutation in the *KDM1A* gene having the SEQ ID NO:1 or SEQ ID NO:2, or in a fragment thereof, in the biological sample of said subject, and/or
(ii) detecting the abnormal expression or activity of the KDM1A protein having the SEQ ID NO:3 in the biological sample of said subject,
wherein the detection of a germline invalidating mutation in the *KDM1A* gene having the SEQ ID NO:1 or SEQ ID NO:2 or in a fragment thereof, or the detection of an abnormal low expression or reduced activity of the KDM1A protein in said sample, is indicative that the subject is predisposed to develop an endocrine disease.

3. The *in vitro* method of claims 1 or 2, wherein said abnormal low expression or reduced activity is due to a reduced expression of the KDM1A protein as compared to control sample, or is due to the expression of a truncated nonfunctional KDM1A protein or is due to the presence of an heterozygous invalidating mutation affecting the KDM1A enzymatic activity.

4. The *in vitro* method of any of claims 1-3, wherein said biological sample is a blood sample.

5. The method of any of claims 1-4, wherein said germline invalidating mutation in the *KDM1A* gene is chosen in the group consisting of: deletions, insertions, point mutations such as mutations affecting splice sites, truncating mutations, frameshift mutations, missense mutation and nonsense mutations.

6. The *in vitro* method of any of claims 1-5, wherein said invalidating mutation in the *KDM1A* gene is chosen in the group consisting of: c.1848dupG, c.352-1G>A, c.1309G>T, c.1737_1738insGA, c.2361T>G, c.2441_2445del, c.952C>T, c.811C>T, and c.386delA.

7. The *in vitro* method of any of claims 1 to 6, wherein said invalidating mutation in the *KDM1A* gene is a deletion of the *KDM1A* locus detected on at least one allele of chromosome 1.

8. An *in vitro* method for the early diagnosis of an endocrine disease in a subject in need thereof, said method comprising the steps of detecting :
- the presence of a germline invalidating mutation in the *KDM1A* gene or in a fragment thereof, or detecting the abnormal expression and/or activity of the KDM1A protein, in a blood sample of said subject,
and
- the presence of another invalidating mutation in the *KDM1A* gene or in a fragment thereof, in a specimen of endocrine tissue of the same subject.

9. An *in vitro* method for the early diagnosis of an endocrine disease in a subject in need thereof, said method comprising the step of :
- detecting the presence of invalidating mutations on the two alleles of the *KDM1A* gene in a specimen of endocrine tissue of said subject,
or
- detecting an abnormal low expression and/or reduced activity of the KDM1A protein in a specimen of endocrine tissue of said subject.

10. The *in vitro* method of claims 8 and 9, wherein said invalidating mutations is found in SEQ ID NO:1 or SEQ ID NO:2, and is preferably chosen in the group consisting of: c.1848dupG, c.352-1G>A, c.1309G>T, c.1737_1738insGA, c.2361T>G, c.2441_2445del, c.952C>T, c.811C>T, and c.386delA.

11. The *in vitro* method of claims 8-10, wherein the presence of said invalidating mutations or abnormal low expression and/or reduced activity indicates that said subject is developing an endocrine disease.

12. The *in vitro* method of claims 9-11, wherein the presence of said invalidating mutations is revealed if the NGS ratio of the mutated alleles in said endocrine tissue is superior to 50%.

13. The *in vitro* method of any of claims 8-12, using the primers of SEQ ID NO:4-5

14. Use of a kit containing the primers of SEQ ID NO:4-5 or antibodies recognizing specifically the KDM1A protein of SEQ ID NO:3, in the methods of claims 1-13.
